# EUROPEAN PATENT SPECIFICATION

(11) **EP 2 935 020 B1**
(45) Date of publication and mention of the grant of the patent: **03.07.2019**
(21) Application number: 13805905.0
(22) Date of filing: 17.12.2013
(51) Int. Cl.: B65B 55/08, A61L 12/08

(54) **DEVICE AND METHOD FOR IRRADIATING PACKAGING CONTAINERS BY ELECTRON BEAM**
VORRICHTUNG UND VERFAHREN ZUR STERILISIERUNG VON VERPACKUNGSBEHÄLTERN MIT ELEKTRONENSTRAHLEN
DISPOSITIF ET PROCÉDÉ POUR STÉRILISER DES RÉCIPIENTS D'EMBALLAGE PAR FAISCEAU D'ÉLECTRONS

(30) Priority: 20.12.2012 EP 12198586; 17.01.2013 SE 1350054; 01.02.2013 SE 1350127; 04.03.2013 SE 1350256; 25.06.2013 SE 1350773
(43) Date of publication of application: 28.10.2015
(73) Proprietor: Tetra Laval Holdings & Finance S.A., 1009 Pully (CH)
(72) Inventor: ÅKESSON, Mats, S-213 63 Malmö (SE); DICKNER, Jonas, S-253 51 Påarp (SE); ZETTERSTRÖM, Håkan, S-241 31 Eslöv (SE)
(74) Representative: Tetra Pak - Patent Attorneys SE
(86) International application number: PCT/EP2013/076871
(87) International publication number: WO 2014/095839

(56) References cited:
- WO-A1-2007/145561
- US-A1- 2012 219 455

## Description

### FIELD OF THE INVENTION

The present invention relates to an irradiation device for irradiating packaging containers with electron beams. The invention also relates to a method.

### BACKGROUND OF THE INVENTION

Within the food industry, it is common practice to pack liquid and partly liquid food products in packaging containers manufactured from a packaging laminate comprising a core layer of paper or paperboard and one or more barrier layers of, for example, polymer material or aluminium foil.

An increasingly common packaging type is the "carton bottle" manufactured in a filling machine in that packaging blanks of the above-described packaging laminate are formed and sealed as a sleeve. Said sleeve is closed in one end in that a top of thermoplastic material is injection moulded directly on the sleeve end portion. The sheets of packaging laminate may be cut from a magazine reel of packaging laminate.

When the top is finished the packaging container is ready to be filled with product through the still open bottom, and then sealed and finally folded. Before the filling operation the packaging container undergoes treatment. If distribution and storage is to be made in chilled temperature the packaging container is disinfected, whereas if distribution and storage is to be made in ambient temperature, the packaging container needs to be sterilized and the product needs to be processed so as to obtain sterility. Sterilization is a term referring to any process that eliminates or kills microbial life, including transmissible agents such as for example fungi, bacteria, viruses and spores, which may be present on a surface of the packaging material or in the product. Applied in the food packaging industry this is generally referred to as aseptic packaging, i.e. packaging sterilized products in sterilized packaging containers, i.e. keeping both the product and the packaging container free from living germs and microorganisms, so that the freshness of the product can be preserved without special cooling requirements, i.e. so that sterility can be maintained inside a packaging container although it is stored in ambient temperature. In the food packaging industry the term commercially sterile is also commonly used. According to Codex Alimentarius Commission ((WHO/FAO) CAC/RCP 40-1993) commercial sterility means " the absence of microorganisms capable of growing in the food at normal non-refrigerated conditions at which the food is likely to be held during manufacture, distribution and storage".

A conventional way of sterilizing a ready-to-fill packaging container is to use hydrogen peroxide, preferably in gas phase.

Another way to sterilize such packaging containers is to irradiate it by means of a low voltage electron beam emitted from an electron beam emitter. An example of linear irradiation by electron beam of ready-to-fill packaging containers is disclosed in the international patent publication WO 2005/002973. The electron beam emitter is cylindrical with an electron exit window positioned at one of the distal ends. The packaging container is lifted to surround the electron beam emitter during the sterilization cycle. Other examples of irradiation of packaging containers, in these cases PET bottles, are described in for example WO 2011/011079 and EP 2 371 397. In the disclosed systems emitters are used having a diameter small enough to be passed through a neck portion of the bottles. US 2012 / 219 455 A1 discloses a device and method for sterilising containers according to the preamble of claims 1 and 12.

### SUMMARY OF THE INVENTION

The present invention relates to an irradiation device for irradiating packaging containers with electron beams according to claim 1.

In one or more embodiments an electron cloud emitted from the first electron beam emitter, upon finalisation of the interior irradiation of the packaging container, is adapted to be partly overlapping the electron cloud emitted from the at least one second electron beam emitter, said electron clouds together forming a combined electron cloud.

In one or more embodiments an opening of the packaging container is at least temporarily positioned within the combined cloud.

In one or more embodiments the first chamber comprises at least first conveying means, and the second chamber comprises at least second conveying means, and the first and second conveying means are separately arranged one on each side of the interface region.

In one or more embodiments the first conveying means is adapted to push the packaging container at least partly through the irradiation lock in the interface region, and release the packaging container when it has been received by the second conveying means on the other side of the irradiation lock.

In one or more embodiments the first electron beam emitter and the packaging container are adapted to perform a mutual relative movement during which the interior irradiation of the packaging container takes place.

In one or more embodiments the first conveying means is adapted to displace the packaging container in relation to the first electron beam emitter between a first position in which the packaging container and the first electron beam emitter are not engaged with each other and a second position in which the packaging container and the first electron beam emitter are fully engaged with each other.

In one or more embodiments it comprises two second electron beam emitters, arranged opposite each other with their electron exit windows facing each other and the interface region, in such a way that the packaging containers can pass in between them.

In one or more embodiments the first electron beam emitter is arranged in the first conveying means, and wherein the first conveying means is adapted to convey the packaging container synchronously with the first electron beam emitter, keeping a longitudinal axis of the first electron beam emitter aligned with a longitudinal axis of the packaging container.

In one or more embodiments the first conveying means is adapted to displace the packaging container from a first chamber in-feed position to an out-feed position, the out-feed position being the interface region, and that the first electron beam emitter is adapted to irradiate the interior of the packaging container at least during a portion of the displacement between the first chamber in-feed position to the out-feed position.

In one or more embodiments the first conveying means is provided with more than one first electron beam emitter.

In one or more embodiments said at least one second electron beam emitter being adapted to irradiate the exterior of the packaging container when passing the packaging container from the first chamber to the second chamber, through the interface region, such that the packaging container reaching the second chamber is fully irradiated on its exterior.

In one or more embodiments the irradiation device is a sterilization device for sterilizing packaging containers.

In one or more embodiments the irradiation lock is an aseptic lock.

In one or more embodiments the second chamber is an aseptic chamber.

In one or more embodiments the aseptic chamber comprises stations in which the packaging container is adapted to be filled with product and sealed.

In one or more embodiments the first chamber is a sterilization chamber.

In one or more embodiments it is arranged in a filling machine.

The present invention also relates to a method for irradiating packaging containers with electron beams according to claim 12.

In one or more embodiments the method comprises the step of finalizing the interior irradiation such that an electron cloud emitted from the first electron beam emitter partly overlaps the electron cloud emitted from the at least one second electron beam emitter, said electron clouds together forming a combined electron cloud.

In one or more embodiments it comprises the step of at least temporarily positioning an opening of the packaging container within the combined cloud.

In one or more embodiments the steps are performed in a filling machine.

In one or more embodiments the steps of irradiating the interior and exterior of the packaging container are steps of sterilizing the interior and exterior of the packaging container.

### BRIEF DESCRIPTION OF THE DRAWINGS

In the following, an embodiment of the invention will be described in greater detail, with reference to the enclosed schematic drawings, in which:
Fig. 1a is a view of a packaging container and an exemplary first electron beam emitter, for sterilizing the interior of the packaging container, in a fully engaged sterilization position,
Fig. 1b is a view of an alternative packaging container and first electron beam emitter,
Fig. 2 is a perspective view of a second electron beam emitter for sterilizing the exterior of the packaging container,
Fig. 3 is a perspective view of a cathode which may be used in the electron beam emitter of Fig. 2,
Fig. 4 is a cross section of the cathode of Fig. 3,
Fig. 5a is a cross sectional view illustrating a first embodiment not of the invention, showing the general principle of the invention,
Fig. 5b is a schematic side view of a second electron beam emitter and its electron cloud,
Fig. 5c is a schematic front view of the second electron beam emitter of Fig. 5b and its electron cloud,
Fig. 5d is a view of a first electron beam emitter and its electron cloud,
Fig. 5e is a view of the volume of the interface region,
Fig. 5f is side view showing two oppositely arranged second electron beam emitters,
Fig. 6a is a perspective view illustrating a second embodiment not of the invention,
Fig. 6b is a top view illustrating portions of the second embodiment not of the invention,
Fig. 6c is a side view of two second electron beam emitters having electron exit windows being inclined relative each other,
Figs. 6d-6g are a cross sectional views of the aseptic chamber, a first electron beam emitter, a second electron beam emitter and a packaging container of the second embodiment,
Fig. 7a is a perspective view of a third embodiment not of the invention,
Fig. 7b is top view illustrating portions of the third embodiment,
Fig. 7c is a side view of a first electron beam emitter, a second electron beam emitter and two packaging containers of the third embodiment,
Fig. 7d is a perspective view of portions of the aseptic chamber of the third embodiment,
Fig. 8 is three top views of the first conveying means of a fourth embodiment not of the invention,
Figs. 9a-9e are perspective views of a fifth embodiment not of the invention, showing different states in a sterilization cycle,
Fig. 10a is a perspective view from one side of a sixth embodiment not of the invention,
Fig. 10b is a perspective view of the sixth embodiment from another side, opposite the one shown in Fig. 10a,
Fig. 10c is a view from above of Fig. 10b,
Fig. 11a is a perspective view of the embodiment of the invention, and
Fig. 11b is a side view of the embodiment of the invention showing the inside of the aseptic chamber.

### DETAILED DESCRIPTION OF A PREFERRED EMBODIMENT

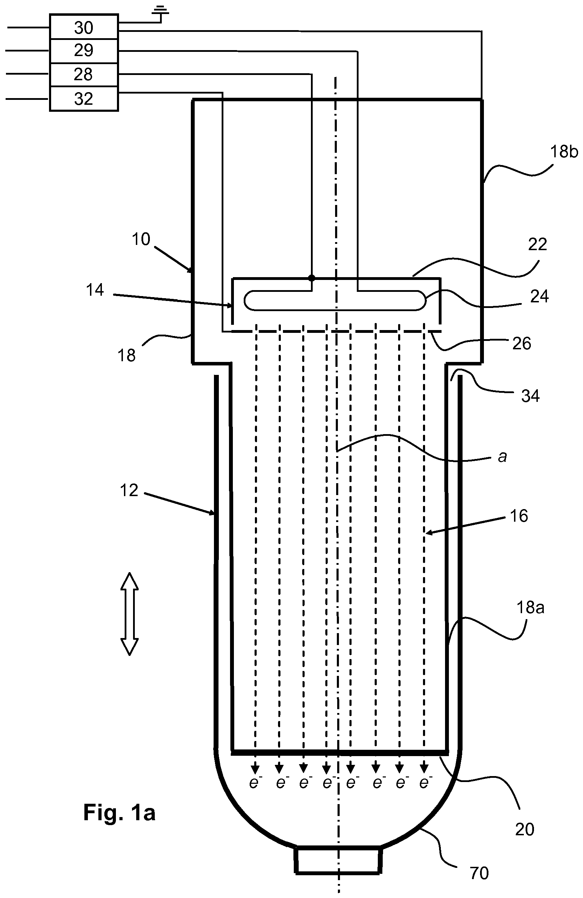

In the following, and with reference to Figure 1a, an exemplary first electron beam emitter 10 for sterilizing, or irradiating, the interior of ready-to-fill packaging containers 12 and the concept of electron beam sterilization or irradiation will be briefly described.

The electron beam emitter 10 comprises an electron generator 14 for emitting a substantially circular electron beam 16 along a path. The electron generator 14 is enclosed in a hermetically sealed vacuum chamber 18. Said vacuum chamber 18 is provided with an electron exit window 20.

The electron generator 14 comprises a cathode housing 22 and a filament 24. Optionally, the electron generator 14 also comprises a control grid 26. In use, an electron beam 16 is generated by heating the filament 24. When an electrical current is fed through the filament 24, the electrical resistance of the filament 24 causes the filament to be heated to a temperature in the order of 2000°C. This heating causes the filament 24 to emit a cloud of electrons. The electrons are accelerated towards the electron exit window 20 by means of a high-voltage potential between the cathode housing 22 and the exit window 20 (being the anode). Further, the electrons pass through the electron exit window 20 and continue towards the target area, i.e. in this case the inside of the packaging container 12.

The high-voltage potential is created by for example connecting the cathode housing 22 and the filament 24 to a power supply 28 and by connecting the vacuum chamber to ground 30. The filament also needs a second connection 29. The first electron beam emitter 10 is generally denoted low voltage electron beam emitter if the voltage is below 300 kV. In the disclosed design the accelerating voltage is in the order of 95 kV. This voltage results in a kinetic (motive) energy of 95 keV in respect of each electron. However, another voltage can be chosen, for example in the interval 75-150 kV. By applying an electrical potential also to the control grid 26 the emission of electrons may be further controlled. If a separate and variable electrical potential is applied to the control grid 26 it makes it possible to use the control grid 26 for active shaping of the generated electron beam. For these purposes the control grid 26 may be electrically connected to a separate power supply 32.

The filament 24 can be made of tungsten. The grid 26, placed between the filament 24 and an electron beam exit window 20 provided with a number of openings and is used for diffusing the electron beam 16 into a more uniform beam, and for focusing the electron beam 16 towards the target area.

The emitter 10 is, as mentioned, further provided with an electron exit window 20. The window 20 can be made of a metallic foil, such as for example titanium, and can have a thickness in the order of 4-12 µm. A supporting net (not shown) formed of aluminum or copper supports the foil from inside the vacuum chamber 18. The electrons are exiting the vacuum chamber 18 through the exit window 20.

In this embodiment the vacuum chamber 18 is made up of two cylindrical bodies 18a, 18b with substantially circular cross sections. An end of the first cylindrical body 18a is provided with the electron exit window 20. The diameter of said first body 18a is small enough to be inserted into the ready-to-fill packaging container 12, the cross section of said first body is dimensioned such that it can be guided through an opening 34 of the packaging container 12. The second body 18b is provided with the electron beam generator14, and the diameter of said second body 18b is larger than the first body 18a. The diameter of the emitted electron beam 16, while still inside the emitter 10, is smaller than the diameter of the first body 18a.

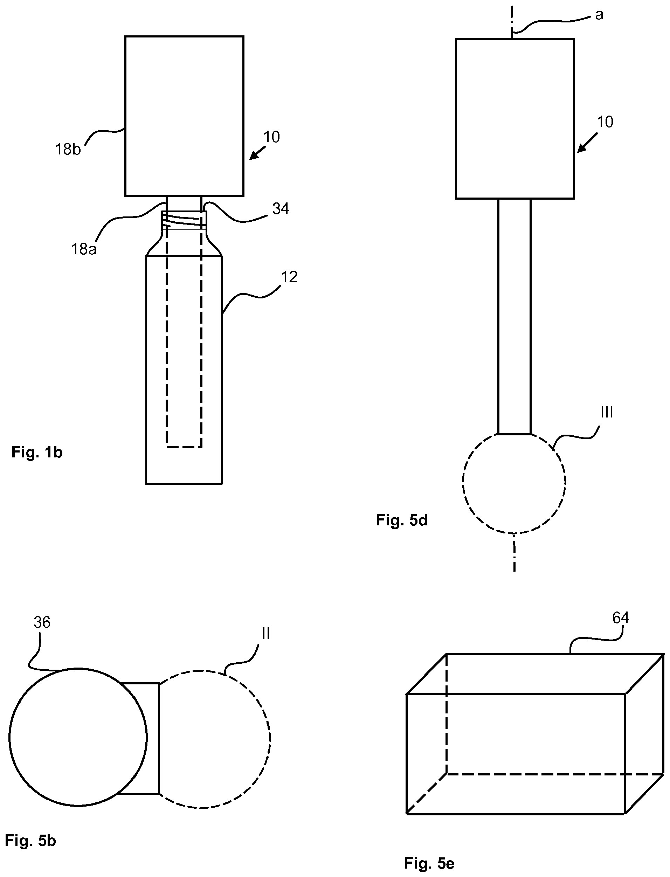

In Figure 1a the opening 34 of the packaging container is an open bottom end, which after filling will be sealed and folded to form a substantially flat bottom surface. It should however be understood that the opening, through which the first electron beam device is received, may in other embodiments be arranged in the top of the packaging container, constituting a neck or spout portion of the packaging container. Figure 1b illustrates such. The neck or spout portion will, after filling, be sealed by for instance a screw cap.

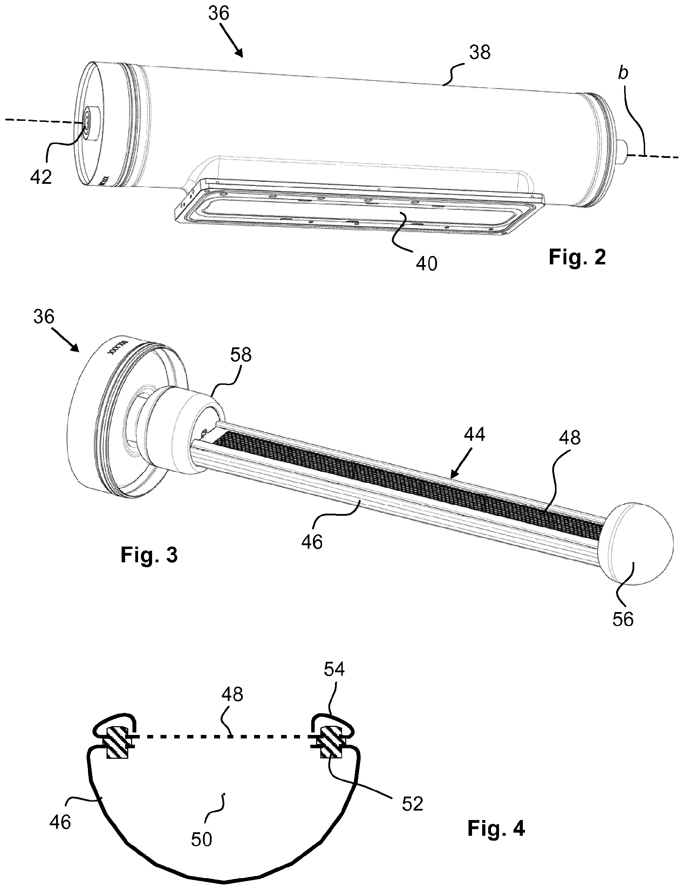

Fig. 2 is a perspective view of an exemplary hermetically sealed second electron beam emitter 36 for sterilizing the exterior of ready-to-fill packaging containers 12. The purpose of the drawing is simply to illustrate the basic components of the emitter, and it should be emphasized that the purpose is not to provide a true constructional drawing or in any other way limit the present invention.

The main component of the electron beam emitter is the tubular body 38, which has an elongate shape along longitudinal axis b. An electron exit window 40 provides an outlet for electrons from the vacuum inside the tubular body 38. The exit window 40 in turn comprises subassemblies not relevant for the present invention, yet having the properties of providing an outlet window for electrons while preserving vacuum inside the body 38. A proximal end of the body 38 comprises an assembly including electrical connections 42.

Fig. 3 shows the emitter 36 without the tubular body 38, and a cathode 44 is shown. The cathode 44 comprises a cathode housing 46, which is also shown in the very schematic cross section of Fig. 4. The cathode housing 46 is formed as a semi-annular shell, the open side of which is covered by a control grid 48. Inside the annular shell of the cathode housing 46 one or more filaments 50 (see Fig. 4) are arranged, extending from a proximal end of the cathode housing 46 to a distal end thereof. In use, an electron beam is generated by heating the filament 50, using a current, and by accelerating the electron towards the electron exit window 40 by means of a high-voltage potential between the cathode housing 46 and the exit window 40 (being the anode). The high-voltage potential is created by for example connecting the cathode housing to a power supply and by connecting the tubular body to ground. By applying an electrical potential also to the control grid 48 the emission of electrons may be further controlled. This can be achieved by connecting the control grid 48 to a separate power supply.

The second electron beam emitter 36 has an accelerating voltage in the order of 95 kV. This voltage results in a kinetic (motive) energy of 95 keV in respect of each electron. However, another voltage can be chosen, for example in the interval 75-150 kV.

The control grid 48 comprises a flat perforated surface comprising a pattern of openings or through-holes for passage of electrons. The open side of the cathode housing 46, carrying the control grid 48, should for obvious reasons be facing the electron exit window 40. The cathode housing 46 and the control grid 48 are mounted together by means of attachment means 52. If there is a difference in electrical potential between the cathode housing 46 and the grid 48 said attachment means 52 are preferably electrical isolator elements. Free longitudinal end portions 54 of the control grid 48 are bent in a direction towards each other, i.e. in a lateral direction being perpendicular to the extension of the longitudinal end portions, to form bulge-like shapes for the formation of electron beam shaping electrodes. Such electrodes are sometimes referred to as "Wehnelt" electrodes. The bulge-like shape will assist in the generation of a smooth predictable electrical field to the benefit of performance of the electron beam emitter. They help shaping the electric field so that the electrons will hit the exit window 40 in an essentially right angle, i.e. in a direction essentially perpendicular to the plane of the exit window 40.

The described cathode is fitted into the electron beam emitter as shown in Fig. 3. The proximal end as well as the distal end of the cathode housing 46 comprises electrical connections as well as physical suspensions for the filament 50. At the distal end this arrangement is housed inside or covered with a dome-shaped cap 54. At its proximal end the cathode housing 46 is suspended to the elongate body and the suspension is encapsulated by an annular cover 58.

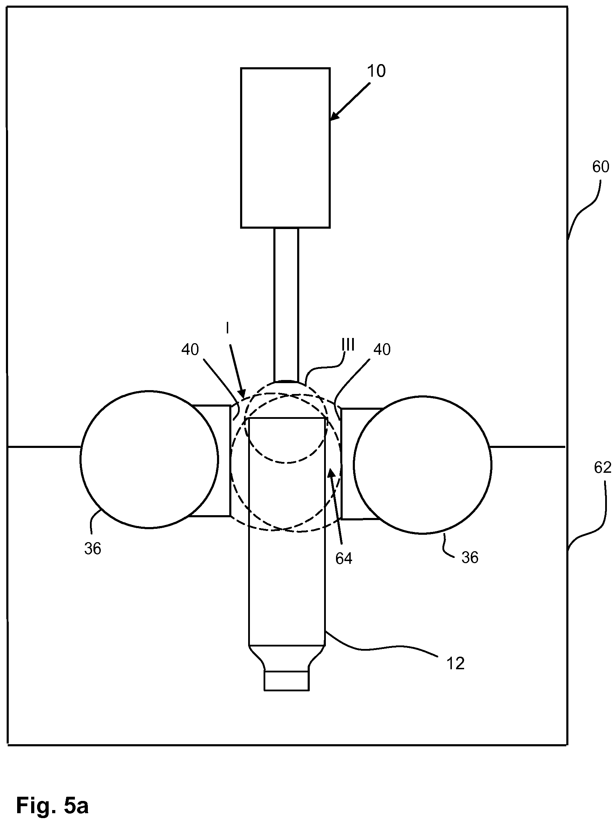

Figures 5a-5f show a first embodiment illustrating the general concept of the invention. In Fig. 5a a first chamber in form of a sterilization chamber of a filling machine is shown. The sterilization chamber is denoted with reference numeral 60. In the sterilization chamber 60 at least one first electron beam emitter 10 is arranged on a first conveying means (not shown). The first electron beam emitter 10 is for example of the type described above in relation to Figure 1a, and it is adapted for sterilization of at least the interior of the packaging container 12. The interior of the packaging container 12 is all inside surfaces of the packaging container. The sterilization chamber 60 is not kept sterile due to the fact that non-sterilized packaging containers 12 are continuously fed into the chamber for the purpose of becoming sterilized. However, near the sterilization chamber 60 there is provided a second chamber in form of an aseptic chamber 62. The aseptic chamber 62 is arranged downstream the sterilization chamber 60, i.e. the packaging containers 12 will first enter the sterilization chamber 60 and then subsequently enter the aseptic chamber 62. During production of packaging containers 12 the environment in the aseptic chamber 62 should be sterile, i.e. free from dirt and microbiological material. The aseptic chamber 62 comprises stations (not shown) in which the packaging container 12 is adapted to be filled with a product, such as for example a beverage, and sealed. Depending on which end of the packaging container that is open, the bottom as shown in Fig. 1a, or the neck as shown in Fig. 1b the sealing station may look different. In the case of filling into an open bottom end the sealing station comprises sealing bars for heatsealing the packaging material. In the case of filling through a spout in a neck region of a packaging container the sealing station instead comprises a capping station. Before the packaging containers 12 have been sealed it needs to be ensured that the environment around them is sterile, that the packaging containers entering the aseptic chamber 62 from the sterilization chamber 60 are sterile, and that no contaminated air is allowed to escape into the aseptic chamber 62 from the sterilization chamber 60.

Between the sterilization chamber 60 and the aseptic chamber 62 there is an interface region 64 through which the packaging containers 12 can pass from the sterilization chamber 60 to the aseptic chamber 62, i.e. the interface region 64 forms an opening 64 between the sterilization chamber 60 and the aseptic chamber 62. Hence, the interface region 64 is an open passage between the sterilization chamber 60 and the aseptic chamber 62. In the vicinity of the interface region 64 there is provided at least one second electron beam emitter 36 adapted for sterilization of at least the exterior of the packaging container 12. The exterior of the packaging container is all surfaces on the outside of the packaging container.

In this specific embodiment there are two second electron beam emitters 36 arranged in the interface region 64. They are arranged opposite each other, with the interface region 64 in between them, such that the packaging containers can pass through it. Further, their electron exit windows 40 are facing the interface region 64 such that the second electron beam emitters 36 are adapted to sterilize the exterior of the packaging containers when those are passed from the sterilization chamber 60 to the aseptic chamber 62 through the interface region 64.

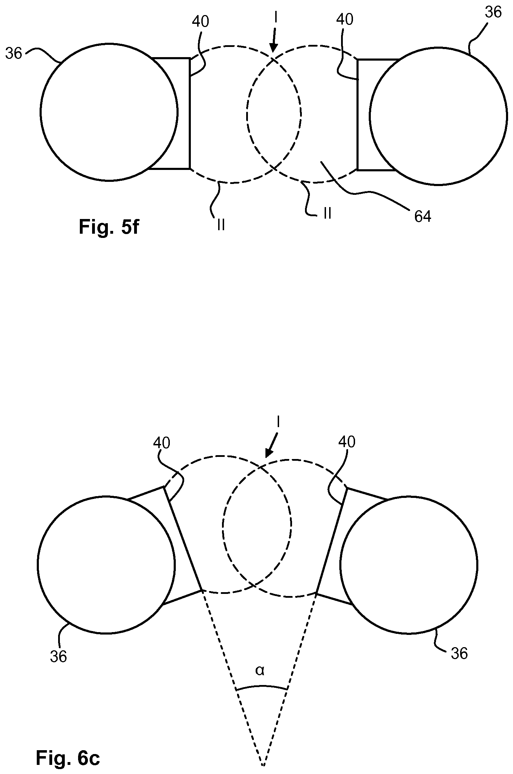

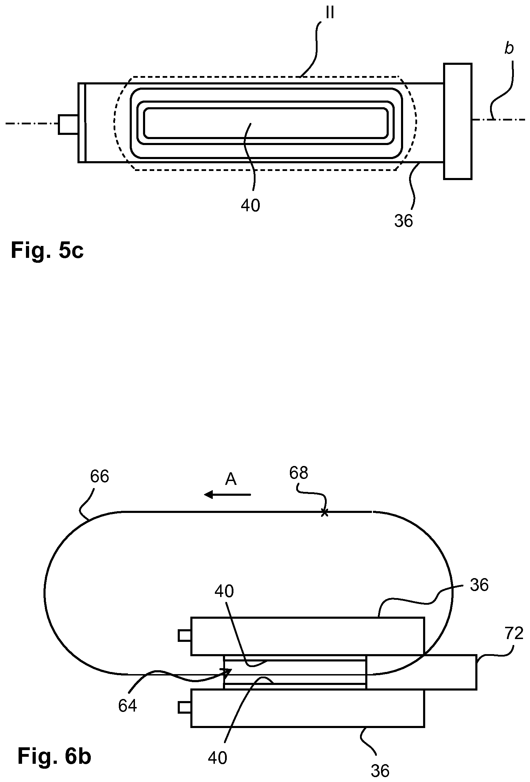

The electron beam generated by each of the second electron beam emitters 36 during operation form a combined electron cloud. The boundary of the cross section of that electron cloud is shown with dashed line I in Figure 5a and Fig. 5f. The electron cloud of one of the second electron beam emitters 36 is shown in Figures 5b, 5c and 5f, and is denoted II. Each cloud II has a substantially circular cross section. The combined electron cloud I fills the interface region 64 forming an irradiation lock, in this case an aseptic lock. The electron cloud I has an extension in three dimensions and forms a volume being large enough to at least cover the interface region 64. Figure 5e illustrates the volume of the interface region 64. The longer sides are approximately as large as the longitudinal extension of the electron exit window 40 of the second electron beam emitter. The volume of the electron cloud I is at least as large as the volume of the interface region 64, i.e. the cloud I at least fills the volume of Fig. 5e. Within the boundaries of the electron cloud I, i.e. the aseptic lock, the electron beams have at least enough energy to kill any microbiological material travelling through the interface region 64 at the same velocity as the velocity of the packaging containers or of any particle or air flow travelling through the same.

In Fig. 2d is shown two second electron beam emitters 36, arranged opposite each other. Typically, for a second electron beam emitter 36 of the above described type, the dose rate at the boundary of the electron cloud II is approx. 400-800 kGy/s. In the centre of the electron cloud the dose rate is higher. The size of the interface region 64 and the energy of the second electron beam emitter will need to be matched for each application such that the electron cloud at least covers the interface region and such that the minimum dose rate of the electron cloud I is enough to kill any microbiological particles passing the interface region 64 at a chosen maximum velocity.

The electron cloud I, i.e. the aseptic lock, is suitable for killing microbiological material within it's direct reach. The innermost interior of the packaging container 12 is not within direct reach, it is "shadowed" by the packaging material of the packaging container. Hence, to ensure that the sterile environment inside the aseptic chamber 62 is not contaminated through the interface region 64 it is important that also the interior of the packaging containers is sterile when entering the aseptic lock, or that any still unsterile portion of the interior of the packaging container is protected by an electron cloud from the first electron beam emitter 10.

Figure 5a shows the case in which the interior of the packaging container 12 is sterile upon entering the aseptic chamber 62. This gives a simple mechanical solution since the first electron beam emitters 10 don't need to enter the aseptic chamber 62. By letting the electron cloud III of the first electron beam emitter 10, upon finalising the interior sterilization of the packaging container 12, at least partly overlap the electron cloud I of the second electron beam emitters, it can then be ensured that every portion of the packaging container 12 reaching the inside of the aseptic chamber 62 has been fully sterilized. In other words, by creating electron clouds I, III being partly overlapping it is ensured that any microbiological material on the inside surface of the packaging container cannot escape to the outside surface, or vice versa, without being killed. Yet, the first electron beam emitters 10 and any conveying means need not enter the aseptic chamber 62.

A cross section of the electron cloud III of the first electron beam emitter 10 is represented by a dashed line III, see Figure 5d. The cross sectional shape is circular, as shown, or droplet-shaped. The electron cloud is axis-symmetrical, around axis *a*, and the cloud volume is thereby spherical (or droplet-shaped). Within the dashed line III the electron beam has at least enough energy to kill any microbiological material travelling through the interface region at the same velocity as the velocity of the packaging containers or of any air flow travelling through the same. Typically, for a first electron beam emitter 10 of the above described type, the dose rate at the boundary of the electron cloud III is approx. 1000-1600 kGy/s. In the centre of the electron cloud the dose rate is higher. The energy of the first electron beam emitter 10 needs to be matched with the sterilization time available and the packaging container size and shape. The first electron beam emitter 10 is adapted to generate an electron cloud III.

It should be pointed out that also the electron cloud III forms an aseptic lock, but an aseptic lock inside the packaging container 12 during sterilization. Although most of the interior sterilization is made in the sterilization chamber 60, not being fully sterile as compared with the aseptic chamber 62, the electron cloud III is able to form an aseptic lock inside the packaging container. No dirt or microbiological material or particle can manage to travel through the cloud III and into the sterilized interior of the packaging container 12. The volume of the electron cloud III of the first emitter 10 covers the opening 34 of the packaging container. Hence, the sterility of the interior, below the electron cloud III, can be assured even during the time when the packaging container 12 is still in the sterilization chamber 60.

Figure 5a shows the moment when the packaging container 12 is on its way through the interface region 64 between the two opposite second electron beam emitters 36. The packaging container 12 is on its way to the first position, meaning that the packaging container and the first electron beam emitter 10 will soon no longer be engaged with each other. Still, the uppermost end of the packaging container, being the open bottom end 34 of the packaging container 12, is still affected by the electron cloud III from the first electron beam emitter 10, although the first electron beam emitter 10 is no longer inside the packaging container 12. At the same time the packaging container 12 has been passed into the electron cloud I of the second beam emitters 36, and most of the exterior of the packaging container 12 has already been sterilized by the electron cloud I of the second beam emitters 36. The packaging container 12 is moved through the interface region 64 with its top and screw cap first. In Figure 5a it can be seen that the electron cloud III of the first electron beam emitter 10 partly overlaps the electron cloud I of the second electron beam emitters 36. The overlapping is created in the area of the uppermost end of the packaging container, i.e. at the open bottom end 34 of the packaging container 12. When the packaging container 12 reaches the aseptic chamber 62 it is fully sterilized.

With Figures 5a-5e the general principle of the aseptic lock has been described. By creating a sterile airflow through the interface region 64, in a direction from the aseptic chamber 62 towards the sterilization chamber 60, the aseptic lock can be sustained even if the second beam emitters 36 are temporarily shut off. Of course the packaging container transport needs to be stopped as well, since no sterilization will take place.

What is not shown in Figure 5a is that the sterilization chamber 60 is provided with first conveying means adapted to push the packaging container 12 at least partly through the aseptic lock in the interface region 64. In the aseptic chamber 62, on the other side of the aseptic lock, there are second conveying means (not shown) that will receive the packaging container 12. When received in the second conveying means the packaging container will be released by the first conveying means. Alternatively, the first conveying means is releasing the packaging container before the second conveying means grip it, having the packaging container falling freely for a moment.

The first conveying means in the sterilization chamber 60 is arranged separately from the second conveying means. The first and second conveying means are separately arranged one on each side of the interface region 64. Hence, there is only necessary to machine-sterilize the second conveying means in the aseptic chamber 62. The first conveying means are gripping the packaging container on the exterior surface. To obtain a fully sterilized packaging container the first conveying means must release the packaging container within the electron cloud I of the second electron beam emitters 36, or within reach of the electron cloud III of the first electron beam emitter 10, to have also the contact surface sterilized. The contact surface is the surface at which the gripping means has gripped the packaging container. Alternatively, the first conveying means are gripping the packaging container on its interior surface. To obtain a fully sterilized packaging container the first conveying means must release the packaging container within the electron cloud III of the first electron beam emitter 10.

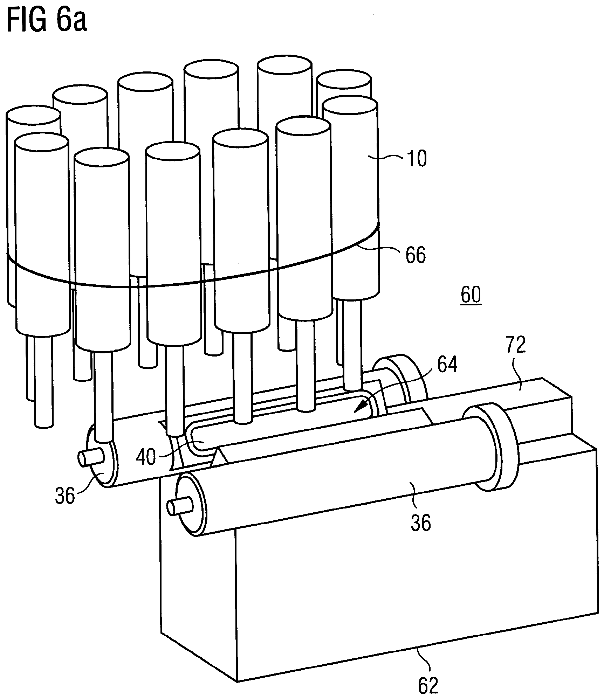

Figures 6a-6h show a more detailed, second embodiment not of the invention. In the following description of the second embodiment some of the reference numerals used for describing the first embodiment will be re-used for similar features.

In this second embodiment several first electron beam emitters 10, of the type described above with reference to Fig. 1, are arranged on first conveying means, see Fig. 6a. The first conveying means comprises a drive belt or chain on which the first electron beam emitters 10 are attached. The belt or chain is just illustrated by line 66. This belt or chain is adapted to convey each packaging container synchronously with a first electron beam emitter 10, keeping a longitudinal axis of the first electron beam emitter aligned with a longitudinal axis of the packaging container, see common longitudinal axis *a* in Fig. 1a. In this embodiment the drive belt or chain 66 of the first conveying means is shaped with two opposite linear portions and two opposite curved portions, see Fig. 6b. The movement of the belt or chain is continuous, but alternatively it is intermittent. The direction of the movement is illustrated by the arrow A.

The first conveying means is adapted to displace the packaging container from a sterilization chamber in-feed position 68 to an out-feed position, the out-feed position being near the interface region 64, at a position slightly above. The in-feed position 68 is located at one of the linear portions of the drive belt or chain 66, whereas the out-feed position is located at the other linear portion, see Fig. 6b. The first electron beam emitter 10 is adapted to sterilize the interior of the packaging container at least during a portion of the displacement from the sterilization in-feed position 68 to the out-feed position.

The first conveying means comprises gripping means, not shown, adapted to grip the packaging container at the in-feed position 68. The packaging container is there started being displaced towards the first electron beam emitter. Since the first electron beam emitter 10 is aligned with the opening 34 of the packaging container 12 the electron beam emitter 10 is received in the packaging container 12. Hence, sterilization of the interior of the packaging container is commenced. Somewhere between the in-feed position 68 and the out-feed position the packaging container has been displaced such that the packaging container is fully engaged with the first electron beam emitter. The fully engaged second position is shown in Fig. 1a. In that position the innermost area of the packaging container 12 may be sterilized, in this case a top portion 70 of the packaging container 12.

The interior sterilization cycle is completed when the packaging container 12 reaches the out-feed position near the interface region 64. When the packaging container 12 reaches said outfeed position, the packaging container is retracted, or has already been retracted from the second position back to the first position. The packaging container is then ready to be fed out from the sterilization chamber and into the aseptic chamber 62.

For achieving the relative vertical movement towards and away from the electron beam emitters the packaging conveying means may comprise a cam curve, servo motor or similar that guides the gripping means in the vertical direction upon rotation of the belt or chain. Further, the gripping means are preferably gripping the packaging container near the opening 34, on the outside surface, by for example a clamping force. However, since the gripping means as such are not the focus of this invention, they will not be described further.

The out-feed position is, as mentioned before, arranged near the interface region 64, slightly above it. On each side of the opening, parallel to the longitudinal direction of the opening, two second electron beam emitters 36 are arranged. They are for example of the type described in relation to Figs. 2-4. They are arranged opposite each other with their electron beam exit windows 40 facing each other and the interface region 64. Either the planes of the electron exit windows 40 are parallel to each other, or slightly inclined in relation to each other as shown in Fig. 6c. In the latter case there is an angle α between the two electron exit windows 40.

Figure 6b shows a top view of the interface region 64, and the two second electron beam emitters 36. Here the electron exit windows 40 are shown as being parallel to each other. The interface region 64, being the only entrance to the aseptic chamber 62 from the sterilization chamber 60, will during operation be totally covered by the electron cloud I of the second emitters. A wall 72, to the right of the electron cloud I, protects the upper portion of the aseptic chamber 62.

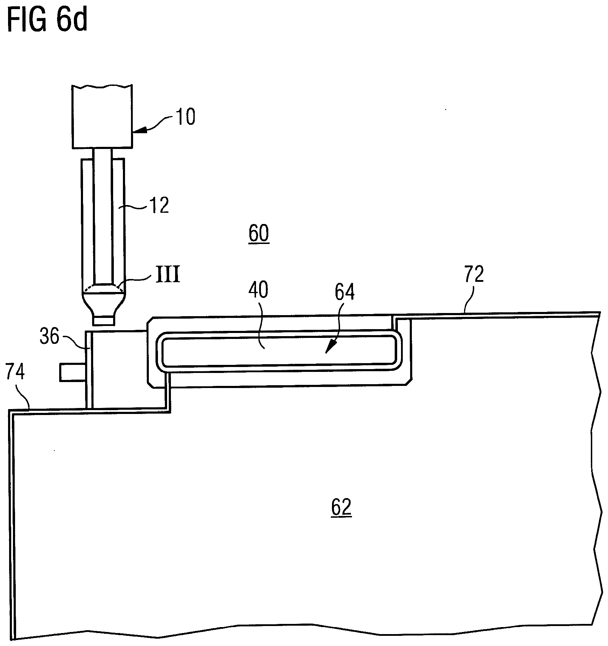

Figures 6d-6g show a cross section through the interface region 64, and a portion of the aseptic chamber 62. Hence, only one second electron beam emitter 36 is shown. The figures further show one first electron beam emitter 10 and one packaging container 12. By means of the Figures 6d-6g the movement of the first electron beam emitter 10 and the packaging container 12 through the interface region 64 will be described.

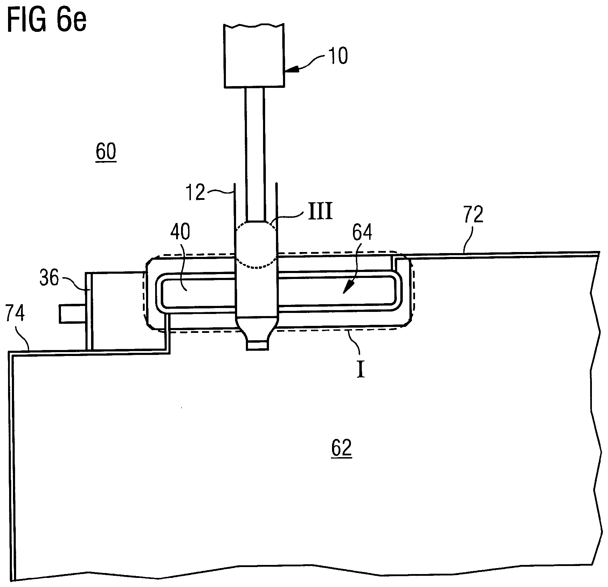

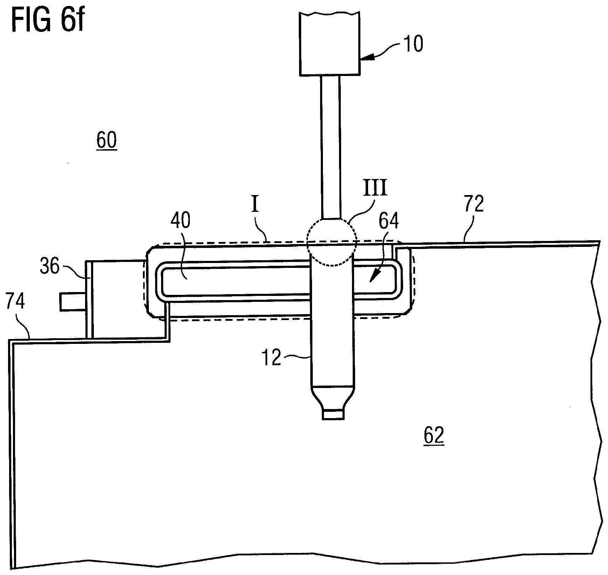

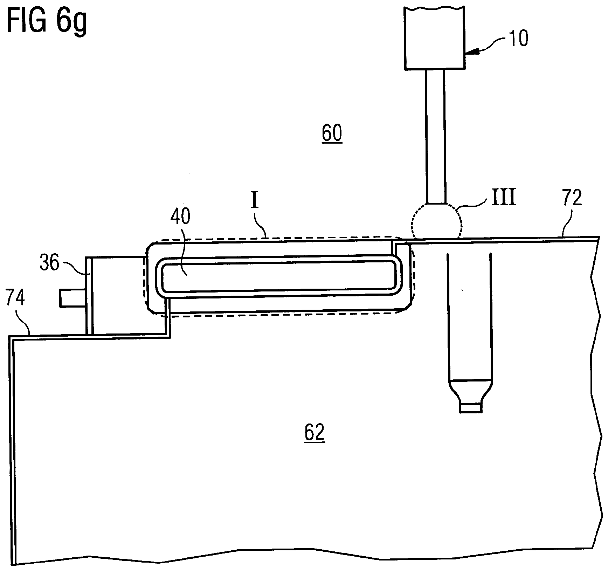

Figure 6d shows the second postion in which the packaging container 12 is fully engaged with the first electron beam emitter 10. The first electron beam emitter 10 and the packaging container 12 have moved from the in-feed position 68 to a position slightly to the left of the interface region 64. Further, as can be seen in the figure, the first electron beam emitter 10 and the packaging container 12 is positioned slightly above the second electron beam emitters 36. Two walls of the aseptic chamber 62 are shown and denoted 72 and 74. In Figure 6e the first electron beam emitter 10 together with the packaging container 12 has moved a distance to the right by means of the belt or chain of the first conveying means. Further, the conveying means has started to vertically displace the packaging container 12 downwards in relation to the first electron beam emitter 10, from the second position to a position closer to the first position. The interior sterilization is on-going. The downward movement has further displaced the packaging container 12 into the electron cloud I of the second electron beam emitters 36, and the packaging container top 70, comprising the screw cap, is already through the interface region 64 and in the aseptic chamber 62. That portion has been sterilized on the outside by the second electron beam emitters 36. In Figure 6f the first electron beam emitter 10 and the packaging container 12 have been moved further to the right. The first electron beam emitter 10 is finalizing the interior sterilization, i.e. only the electron cloud III of the first electron beam emitter 10 is engaged with the opening 34 in the bottom end of the packaging container 12. In this moment the electron cloud III of the first electron beam emitter 10 is partly overlapping the electron cloud I of the second electron beam emitters. The gripping means of the first conveying means, positioned in the sterilization chamber 60, has released the packaging container 12, i.e. it is no longer gripping the packaging container. The gripping means is not passing the interface region 64 in this embodiment. Instead, the second conveying means, not shown, of the aseptic chamber 62 is within reach of the packaging container 12 and has taken over the gripping of the packaging container. Finally, Figure 6g shows a moment in which the packaging container 12 is fully sterilized and has been conveyed to the right in the figure by means of the second conveying means. The upper wall portion 72 of the aseptic chamber 62 is protecting the packaging container 12. On the other side of said wall 72 the first electron beam emitter 10 is conveyed, by the first conveying means, back to the in-feed position. On its way back to the in-feed position 68 the first electron beam emitter 10 moves past a sensor (not shown) for measurement of at least one dose control parameter of the electron beam, such as for example the dose rate (kGy/s).

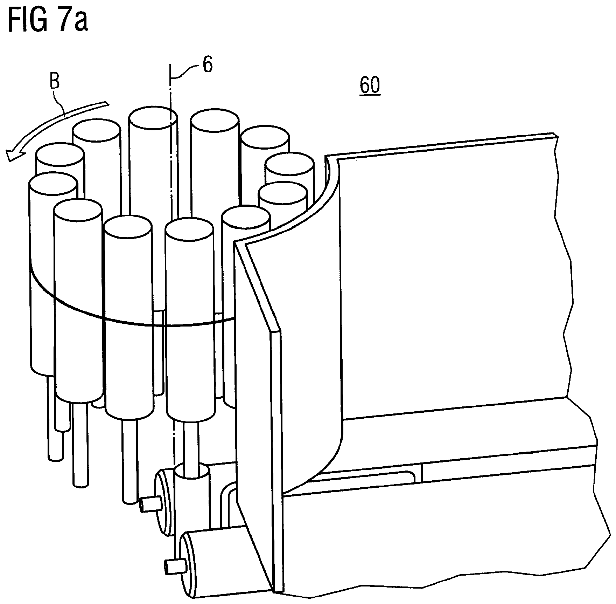

Figures 7a-7d show a more detailed, third embodiment not of the invention. In this third embodiment some of the reference numerals used for describing the second embodiment will be re-used for similar features. Only the differences between the third and second embodiment will be described.

Figure 7a shows a sterilization device provided with several first electron beam emitters 10 of the type used for interior sterilization of packaging containers 12. Such first electron beam emitters 10 were described above with reference to Fig. 1a.

The first electron beam emitters 10 are provided to first conveying means. The first conveying means comprises a rotatable carrier, shown as a line 76. The rotatable carrier is, in this embodiment, a wheel and is rotatable round a centre shaft, here illustrated as a dash-dotted line b. The direction of the rotation is illustrated by the arrow B and the rotatable movement is continuous. The first emitters 10 are equally distributed to the periphery of the carrier 76, and are fixed to the carrier so that they are being carried along when the carrier 76 rotates. The transportation of the packaging containers 12 is made in a direction transverse to the longitudinal extension of the emitters 10.

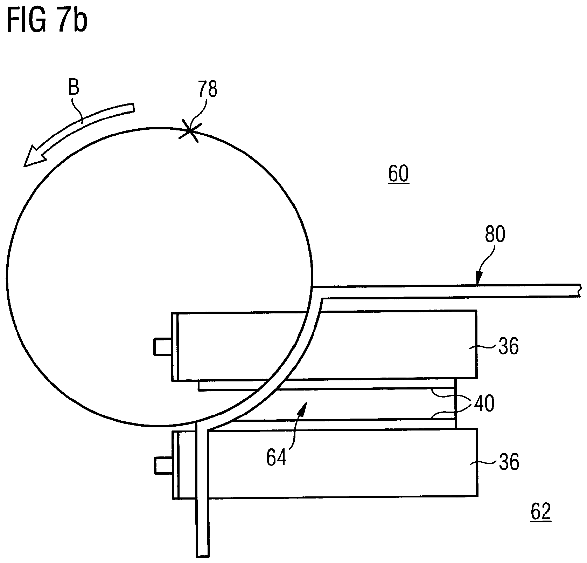

The circle of Figure 7b shows, very schematically, the transportation path of the packaging container and the first electron beam emitter. The first conveying means, not shown, is adapted to convey the packaging container from an in-feed position 78 in the sterilization chamber 60 to an outfeed position, said out-feed position being close to the interface region 64 towards the aseptic chamber 62. The conveying of the packaging container is made synchronously with the carrier revolution movement and in alignment with the electron beam emitter 10. A longitudinal centre axis of the packaging container is aligned with a longitudinal centre axis of the electron beam emitter, see common axis *a* in Fig. 1a. Further, the opening 34 in the packaging container 12 is concentric with the longitudinal centre axis of the packaging container.

The first conveying means is being further adapted to vertically displace the packaging container 12 in relation to the first electron beam emitter 10. In the embodiment shown the electron beam emitters 10 are arranged stationary in the carrier 76 and cannot move towards the packaging container. Due to the heavy weight, the fragile electron exit window and the high voltage connections it is an advantage to have the first electron beam emitters 10 stationary, and move the packaging containers 12.

The first conveying means displaces the packaging container 12 between a first position in which the packaging container 12 and the electron beam emitter 10 are not engaged with each other and a second position in which the packaging container 12 and the electron beam emitter 10 are fully engaged with each other. When the packaging container 12 and the electron beam emitter 10 are engaged the packaging container 12 has been raised to a position in which it surrounds the electron beam emitter 10 and the electron beam emitter 10 is sterilizing the interior of the packaging container. When they are not engaged the packaging container is positioned underneath the electron beam emitter, i.e. the packaging container has not started to surround the emitter 10, or has just been displaced down from the engaged position. At the in-feed position 78 and out-feed position the packaging container 12 is positioned in the first position, i.e. not in engagement with the electron beam emitter 10.

At the in-feed position 78 the packaging containers 12 are supplied to the sterilization device in the sterilization chamber 60. Each packaging container 12 is aligned with a corresponding electron beam emitter 10 and is gripped by means of gripping means (not shown). The gripping means is comprised in the first conveying means. The gripping means are attached to a drive belt or chain (not shown). The gripping means preferably grips the packaging container 12 in the top portion 70.

When the carrier 76 of the first conveying means rotates, so that the electron beam emitter 10 and packaging container 12 rotates from the in-feed position 78 to the interface region 64, the gripping means displaces the packaging container 12 towards the first electron beam emitter 10 for performing interior sterilisation.

Figures 7a and 7b further show two second electron beam emitters 36 for example of the type described in relation to Figs. 2-4. Similar to the arrangement of the two second electron beam emitters of the second embodiment, these two electron beam emitters 36 are arranged one on each side of the interface region, parallel to the longitudinal direction of the opening. They are arranged opposite each other with their electron beam exit windows 40 facing each other and the interface region. The planes of the electron exit windows are parallel to each other, but may alternatively be inclined as described in relation to Fig. 6c.

Figure 7a further shows a part of the aseptic chamber 62. The only entrance to the aseptic chamber 62 from the sterilization chamber 60 is the interface region 64. Some of the walls, together denoted 80, needed towards the sterilization chamber 60 are shown in figure. It is however to be understood that not all necessary walls are shown, and that the aseptic chamber 62 is closed except for the opening 64 towards the sterilization chamber 60 and a packaging container outlet (not shown) for out-feed of finished packaging containers 12.

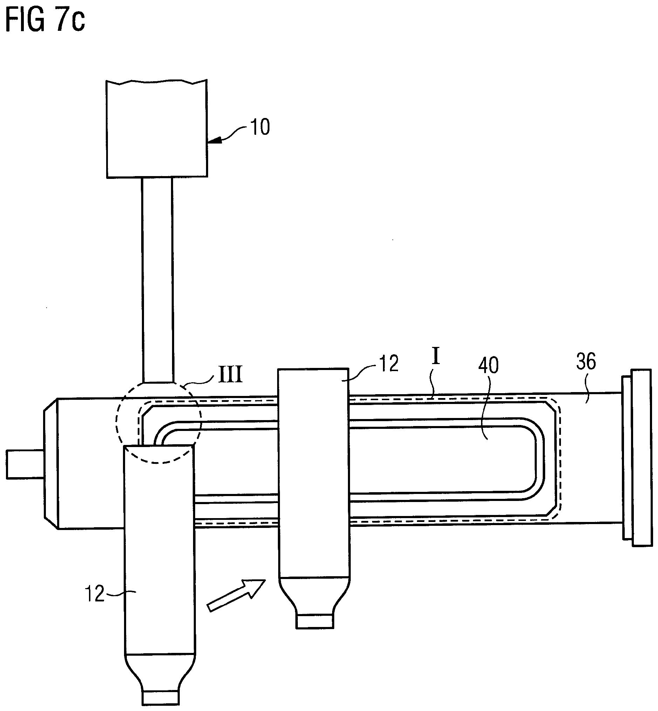

Figures 7c show a cross section through the opening in the interface region 64. Hence, only one second electron beam emitter 36 is shown. The figure further show two subsequent packaging containers 12 and one first electron beam emitter 10.

By means of the Figures 7a-7d the movement of one first electron beam emitter 10 and the packaging container 12 through the interface region 64 will be described.

When the packaging containers 12 has reached the position shown in Figure 7a it has been conveyed from the in-feed position 78 and almost to the out-feed position, i.e. the interface region 64. The packaging container 12 is being displaced downwards by the gripping means, from the second position to the first position, and the interior sterilization is on-going.

In Figure 7c the left packaging container 12 has been displaced further downwards and the interior sterilization is about to be finalised. Only the electron cloud III of the first electron beam emitter 10 is engaged with the opening 34 in the bottom end of the packaging container 12. At the same time the packaging container 12 as well as the first electron beam emitter 10 has been conveyed closer to the out-feed position, i.e. to the right in the figure and i.e. closer to the opening 64 towards the aseptic chamber 62. When the left packaging container 12 is moved to the right into the electron cloud I of the second electron beam emitters 36 the electron cloud III of the first electron beam emitter 10 will be partly overlapping the electron cloud I of the second electron beam emitters 36. Upon further displacement to the right, see the right packaging container 12, the gripping means will raise the packaging container so that it will move through the opening and the electron cloud I of the second electron beam emitters 36, i.e. through the aseptic lock, and become sterile on its outside surface. Before the top portion 70 of the packaging container 12 is moved into the aseptic lock/interface region 64 the gripping means of the first conveying means (not shown) releases the packaging container, and second conveying means (not shown) in the aseptic chamber 62 grips the packaging container and takes over the conveyance. In Figure 7c no walls of the aseptic chamber is shown.

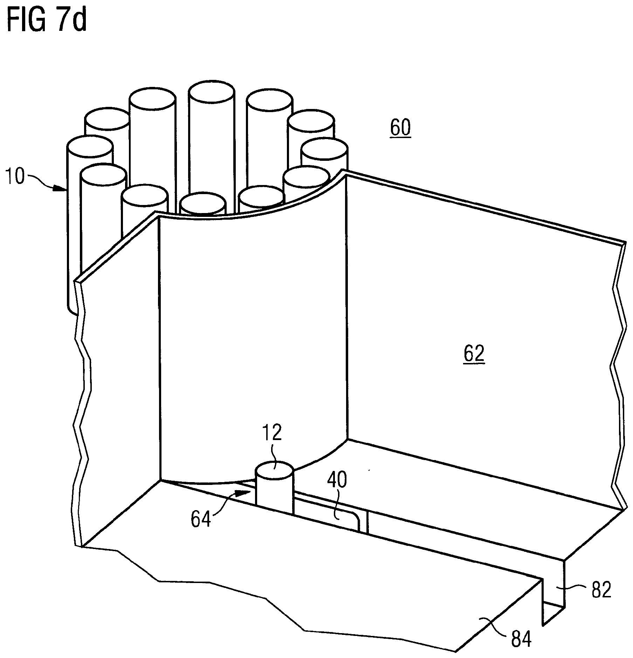

Figure 7d shows the interface region 64, i.e. the passage into the aseptic chamber 62 and a portion of a packaging container 12 being raised through the interface region. The figure also shows the walls 80 around the opening 64 as well as a ditch 82 in the lowermost wall portion 84 for receiving the top portion 70 of the packaging container 12 upon further movement up through the opening and into the aseptic chamber 62.

The invention comprises a method of sterilizing packaging containers with electron beams in a filling machine. The method has to a large extent already been described in relation to the various embodiments, and will only be summarized in the following. The method comprises the steps of sterilizing at least the interior of the packaging container with a first electron beam emitter arranged in a sterilization chamber, and sterilizing at least the exterior of the packaging container with a second electron beam emitter. The step of sterilizing the exterior of the packaging container is performed in an interface region, said interface region forming an opening from the sterilization chamber to an aseptic chamber. An electron cloud emitted from the second electron beam emitter during operation is adapted to form an aseptic lock at least covering the opening in the interface region. The method further comprises the step of finalizing the interior sterilization such that an electron cloud emitted from the first electron beam emitter partly overlaps the electron cloud emitted from the second electron beam emitter.

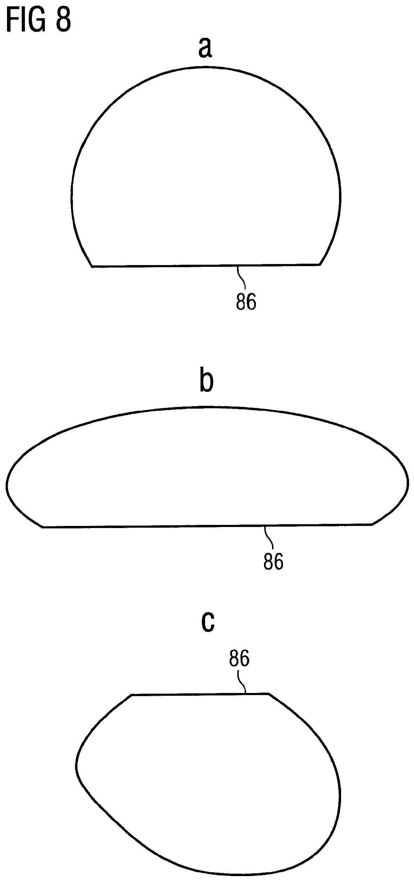

A fourth embodiment not of the invention is shown in Fig. 8. The fourth embodiment is similar to the embodiment shown and described in accordance with Figs. 6a and 6b, except for the shape of the drive belt or chain of first conveying means. In Fig. 6a the drive belt or chain of the first conveying means is shown as a line 66, being shaped with two opposite linear portions and two opposite curved portions. It is to be understood that this shape is not the only possible shape. Fig. 8 show three exemplary alternative shapes denoted *a*, *b* and *c*. A portion, denoted 86, of the drive belt or chain, is preferably linear or straight. That is the portion passed the second electron beam emitters 36. The rest of the belt or chain may have any shape such as for example partly or semi-circular, as *a*, or partly or semi elliptic, as b, or just following any suitable curve, of which an example is shown as c.

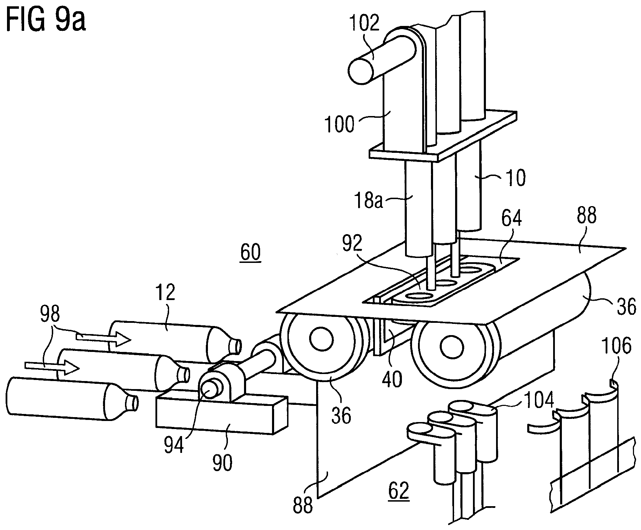

A fifth embodiment not of the invention is shown in Figs. 9a-9e. This is an embodiment working according to the same sterilization principle as the previously described embodiments, but having a very different design. To the extent possible the same reference numerals as in the previously described embodiments will be used for similar features.

In each figure, showing different positions of a sterilization cycle, as will be described below, only a set of three packaging containers 12 side by side will be present, for ease of understanding. It should however be understood that, in reality, there is a "continuous" flow of packaging containers throughout the sterilization device and that therefore a plurality of packaging containers are present in the sterilization device simultaneously and present in different positions in the sterilization cycle.

In the fifth embodiment is an intermittent version of the invention, meaning that the packaging containers are fed intermittently through the sterilization device. However, it should be understood that the packaging containers may be fed with a continuous motion both before and after the sterilization device, i.e. throughout the rest of the filling machine.

In the fifth embodiment a number of first electron beam emitters 10 are arranged side by side, i.e. their longitudinal axes are substantially parallel, and as in the previously described embodiments the first electron beam emitters 10 are adapted for sterilization of at least the interior of a packaging container 12. In this embodiment three first electron beam emitters 10 are arranged for sterilizing three packaging containers 12 at a time. In alternative embodiments the number of first electron beam emitters may be smaller or larger depending on the desired capacity of the filling machine. Further, two second electron beam emitters 36 are provided adapted for sterilization of at least the exterior of the packaging container 12. Similar to the arrangement of the two second electron beam emitters of the previous embodiments, electron exit windows 40 of the two second electron beam emitters 36 are facing an interface region 64, said interface region 64 forming an opening from a sterilization chamber 60 to an aseptic chamber 62. They are arranged one on each side of the interface region 64, parallel to the longitudinal direction of the opening, opposite each other with their electron beam exit windows 40 facing each other and the interface region 64. The planes of the electron exit windows 40 are parallel to each other, but may alternatively be inclined as described in relation to Fig. 6c.

In the sterilization chamber 60 the first electron beam emitters 10 are adapted to sterilize the interior of the packaging container 12.

The environments in the sterilization and aseptic chambers are separated by walls 88, of which only two are partly shown in Fig. 9a. It is however to be understood that the only opening between the chambers 60, 62 is the interface region 64 between the second electron beam emitters 36. Although not visible, the aseptic chamber 62 is encapsulated by walls to be able to maintain a sterile environment.

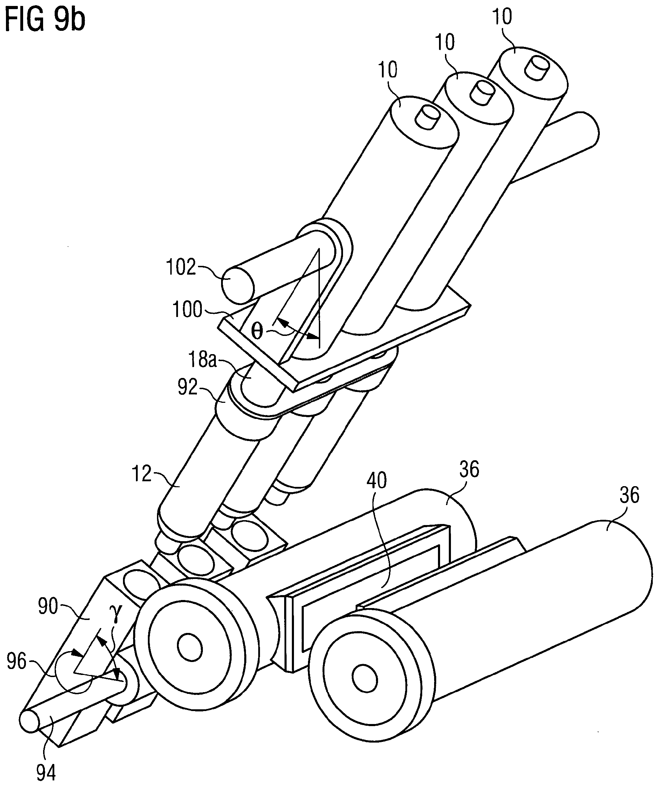

In the sterilization chamber 60 first conveying means is handling the packaging containers. The first conveying means comprises a first set of carriers 90 and a second set of carriers 92. The carriers 90 of the first set are adapted to receive one packaging container 12 each. Said carriers 90 are suspended on a pivot axle 94 around which they can pivot between a packaging container load position and a packaging container un-load position. The load position is shown in Fig. 9a and the un-load position is shown in Fig. 9b. In this exemplary embodiment the carriers 90 are positioned in a horizontal direction in the load position. When the carriers 90 have been pivoted to the un-load position they have been rotated clockwise around the pivot axle 94 to a position where they are angled an angle γ, less than 90°, with regard to the horizontal direction. The rotational movement is shown as arrow 96. The rotation back to the load position is made counter clockwise.

Further, the carriers 90 of the first set are arranged to be displaced along the pivot axle 94 between a separated position and a gathered position. The separated position is shown in Fig. 9a and the gathered position is shown in Fig. 9b. In the load position the first set of carriers 90 are in the separated position and in the un-load position said carriers 90 are in the gathered position.

In the load position packaging containers 12 are loaded in the carriers 90 from feeding conveyors. The feeding conveyors are just illustrated as arrows 98 in Fig. 9a. The carriers then pivot around the axle 94 into the un-load position, at the same time the carriers 90 move from the separated position to the gathered position. From this position the packaging containers 12 are fetched by the second set of carriers 92 of the first conveying means. This is shown in Fig. 9b. The carriers 92 of the second set are arranged together with the first electron beam emitters 10. Each carrier 92 is adapted to fetch a packaging container 12 from the first set of carriers 90 and displace it along the portion 18a of the first electron beam emitter 10 in order for the first electron beam emitter 10 to sterilize the packaging container 12.

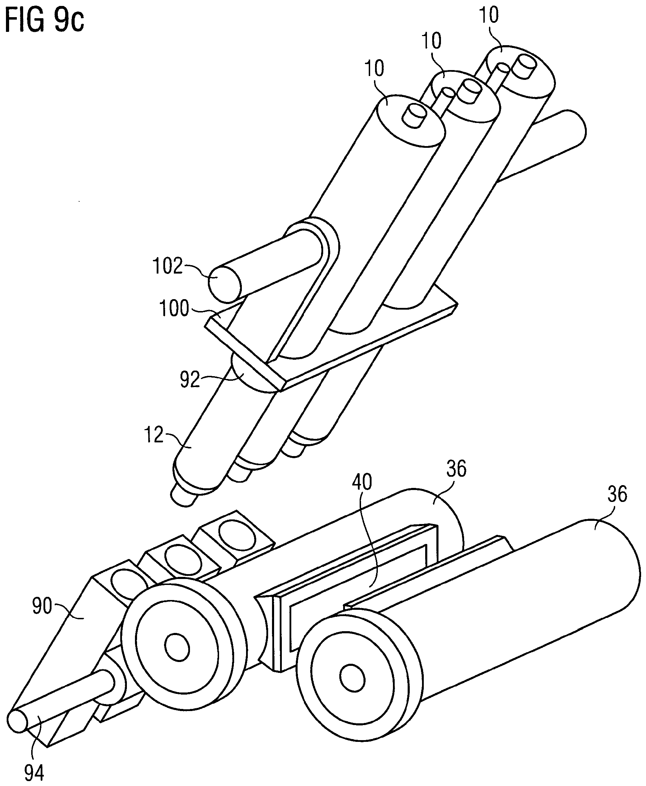

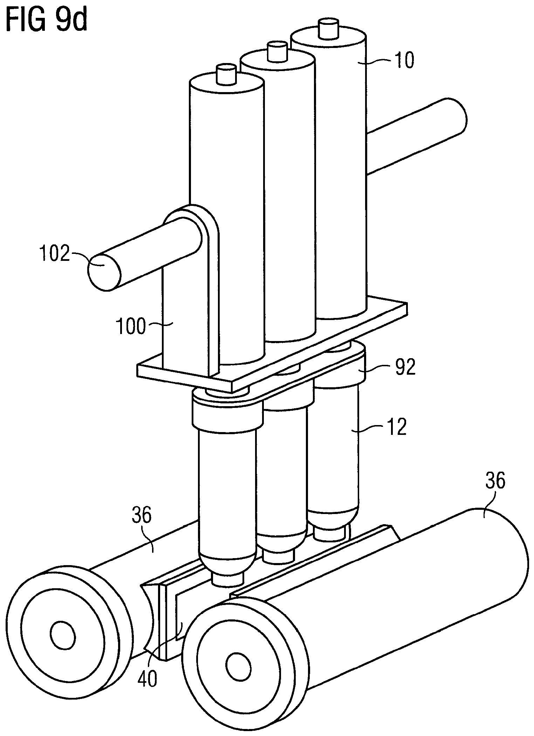

The second set of carriers 92 is movably arranged in a suspension device 100, which suspension device 100 is provided with a pivot axle 102 and in which suspension device 100 the first electron beam emitters 10 are fixedly attached, i.e. stationary. By means of the pivot axle 102 the suspension device 100 is adapted to rotate the first electron beam emitters 10 and the second set of carriers 92 between a packaging container load-position, shown in Figs. 9b and 9c, and a packaging container un-load position, shown in Figs. 9a and 9d. In the load position of the suspension device 100 the longitudinal axes of the first electron beam emitters 10 are aligned with the longitudinal axes of the packaging containers 12 held by the first set of carriers 90 being in their un-load position. In the un-load position of the suspension device 100 the first electron beam emitters 10 together with the second set of carriers 92 are positioned in the vertical direction. The load position of the suspension device 100 is angled an angle θ to the vertical direction of the un-load position of the suspension device 100. The rotation of the suspension device 100, from the load position to the un-load position, is made in the counter clockwise direction whereas the rotation from the un-load position to the load position is made in the clockwise direction.

In Fig. 9b the packaging containers 12 have just been fetched from the first set of carriers 90, and in Fig. 9c the packaging containers 12 have been fully engaged with the first electron beam emitters 10. Sterilization of the packaging container inside surface is on-going.

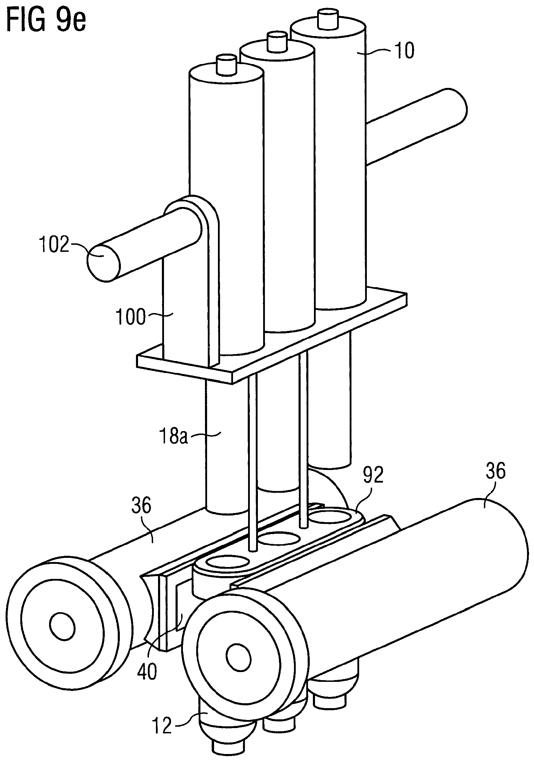

Fig. 9d shows one of the final steps in the sterilization cycle. The suspension device 100 has pivoted the first electron beam emitters 10 together with the packaging containers 12 to the un-load position. The packaging containers 12 are positioned in the fully engaged position on the first electron beam emitters 10 and are positioned above the interface region 64 being the opening to the aseptic chamber 62. The first set of carriers 90 then displace the packaging containers 12 downwards along the electron beam emitters 10 and through the interface region 64. When the packaging containers 12 are passing through the interface region 64 the packaging containers 12 are sterilized on their outsides surfaces. When the packaging container 12 has reached a position when its opening 34 is beneath the electron exit window 20 of the first electron beam emitter 10, i.e. when the first electron beam emitter 10 is about to not being engaged with it, or is no longer engaged with it any longer, the electron cloud III of the first electron beam emitters 10 are at least partly overlapping the electron cloud 1 of the second electron beam emitters 36. This position is shown in Fig. 9e. At this point the first set of carriers 90 release the packaging containers 12 and the packaging containers 12 are received inside the aseptic chamber 62 firstly by an intermediate conveying means 104 and secondly by a second conveying means 106, see Fig. 9a for 104 and 106. When received in the intermediate conveying means 104, the intermediate conveying means 104 is lowered to the level of the second conveying means 106. Then, the intermediate conveying means 104 rotates the packaging containers into grippers in the second conveying means 106. The second conveying means 106 conveys the packaging containers to a filling station for filling product into the sterilised packaging container.

The second set of carriers 92 can grip the packaging containers 12 on packaging container outside surface, as shown in the present embodiment, or on the packaging container inside surface, or a combination of both. The displacement up and down of the second set of carriers 92 may be made by for example linear motors positioned in between the three first electron beam emitters 10. The carriers 90 of the first set have a design in the form of containers for receiving the packaging containers therein. This is an exemplary design, and of course many other designs are possible.

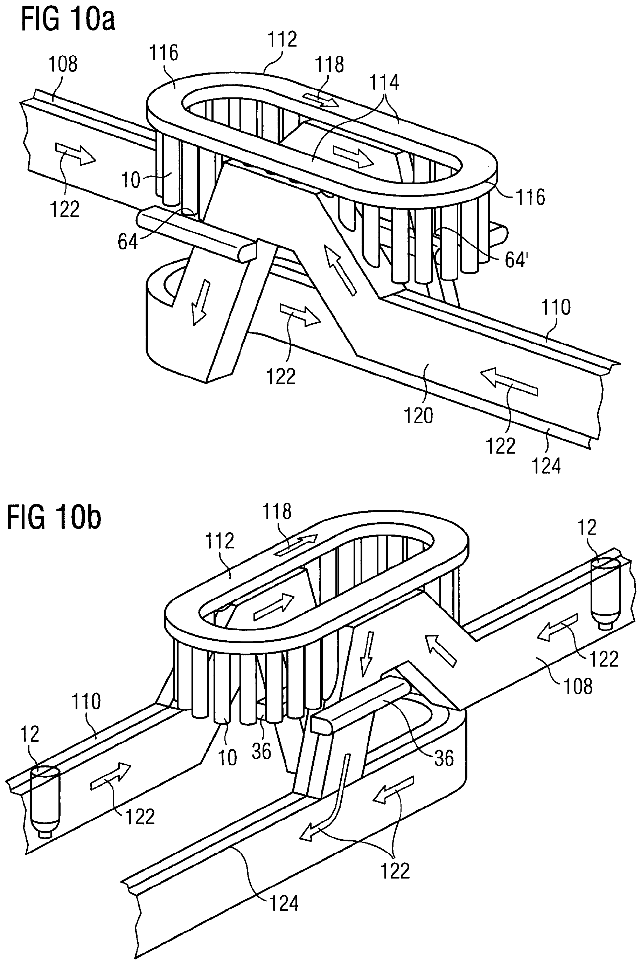

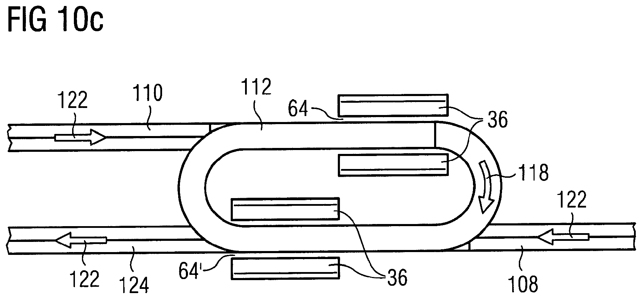

A sixth embodiment is shown in Figs. 10a, 10b and 10c and it is working according to the same sterilization principle as the previously described embodiments. The main difference lies in the packaging container feeding. In this sixth embodiment the first conveying means comprises two packaging container conveying means 108, 110 feeding packaging containers through the sterilization chamber.

To some extent the same reference numerals as in the previously described embodiments will be used for similar features.

The first conveying means further comprises a first electron beam emitter conveying means 112 for conveying a plurality of first electron beam emitters 10. The first electron beam emitter conveying means 112 comprises a belt or chain formed as a race track or an oval, having two opposite, parallel, straight portions 114 bound together by two opposite curved portions 116. The belt or chain is rotating in the clockwise direction as in relation to Fig. 10a, and illustrated by arrow 118. Further, there are two sets of second electron beam emitters 36. A first set of second electron beam emitters 36 is forming the first entrance to the aseptic chamber. They are positioned in parallel and opposite each other forming a first interface region 64 in between. Further, they are positioned along one of the straight portions 114 of the first electron beam emitter conveying means 112 so that the first electron beam emitters 10 can pass in between the second electron beam emitters 36. Similarly, a second set of second electron beam emitters 36 is forming the second entrance to the aseptic chamber. The second electron beam emitters 36 of the second set are positioned in parallel and opposite each other forming a second interface region 64' in between them. Further, they are positioned along the other one of the straight portions 114 of the first electron beam emitter conveying means 112 so that the first electron beam emitters 10 can pass in between the second electron beam emitters 36.

In the figures the packaging container movement is shown as a fictive band 120 having a height similar to the packaging container height. An exemplary packaging container 12 is shown on the band 120 in a couple of positions to illustrate this, see Fig. 10b. Several arrows 122 show the direction of the movement.

In the sterilization chamber the packaging container movement, illustrated as the fictive band 120, is made by the previously mentioned packaging container conveying means 108, 110. The conveying means are not shown. They comprise lifting means (not shown) for lifting the packaging containers 12 towards the first electron beam emitters 10 for the inside surface sterilisation. The lifting is taking place along the straight portions 114 of the first electron beam emitter conveying means 112. The fully engaged position between a packaging container 12 and a first electron beam emitter 10, in which the first electron beam emitter is sterilizing the innermost part of the packaging container, see Fig. 1a, is reached approximately half way along the straight portion 114. Then the packaging container 12 is lowered with respect to the first electron beam emitter 10 and passes the interface region 64, 64' between the second electron beam emitters 36. The packaging containers 12 are sterilised on their outside surface. The packaging container 12 is lowered such that the electron cloud III of the first electron beam emitter 10 is at least partly overlapping the electron cloud I,II of the second electron beam emitters 36, as described in relation to the other embodiments. When having passed the interface region 64, 64' the packaging container reaches the aseptic chamber and has become fully sterilised, both inside and outside. The aseptic chamber is situated below the sterilization chamber in the figures.

In the figures there is not shown any walls separating the sterilization chamber and the aseptic chamber, but it should be understood that the only open passages between them is formed by the first and second interface regions 64, 64' as have been shown in the other embodiments.

At one point in time, when the packaging container 12 is situated in the interface region 64, 64', the packaging container 12 is released by the packaging container conveyors 108, 110 and is instead received by a second conveying means 124 in the aseptic chamber. That second conveying means 124 receives packaging containers from both interface regions 64, 64' and conveys them to filling stations etc. The second conveying means 124 in the aseptic chamber is separate from the packaging container conveying means 108, 110 of the first conveying means in the sterilization chamber. They are separated at the interface regions 64, 64', such that they are not extending into each other's chambers, as described in the previous embodiments. However, it should once again be noted that the entire packaging container movement is shown as a band 120 in the figures, and in the figures the band 120 is illustrated as being continuous in the interface regions 64, 64'.

One advantage with this embodiment is that each first electron beam emitter sterilizes two packaging containers per one rotation of the first electron beam emitter conveying means, which increases the utilization of the first electron beam emitters.

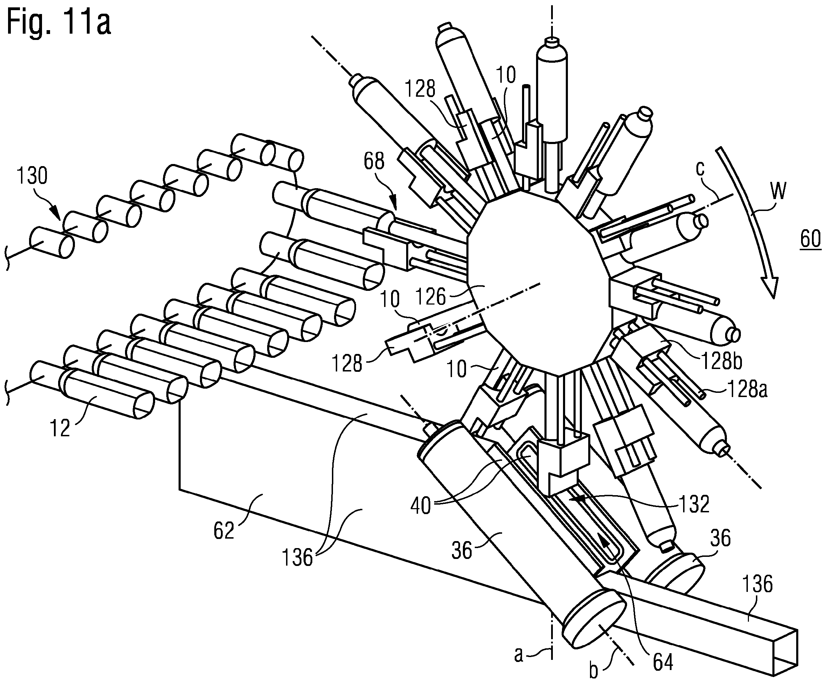

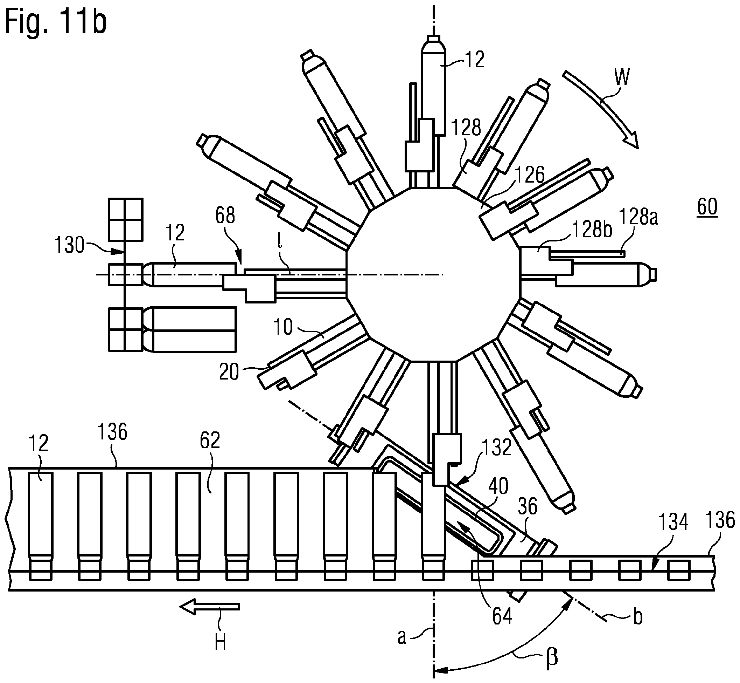

Figs. 11a and 11b show the embodiment of the invention. The seventh embodiment is based on the same general principle as the previously described embodiments.

To some extent the same reference numerals as in the previously described embodiments will be used for similar features.

In this embodiment the first conveying means comprises a carrier wheel 126. The carrier wheel 126 is positioned in the sterilization chamber 60. The first electron beam emitters 10 are arranged as spokes on the carrier wheel 126. Their respective longitudinal axis is radially directed in relation to a centre of the carrier wheel 126. A centre axis of the wheel is shown as dash-dotted line c in Fig. 11a. The first electron beam emitters 10 each have an electron exit window 20 arranged such that a plane aligned with the window surface is provided transverse to the longitudinal axis of the first electron beam emitter 10, see Fig. 1a. The electron exit window 20 is positioned in the outer end of each spoke, i.e farthest from the centre of the carrier wheel 126, see Fig. 11b.

In this example twelve first electron beam emitters 10 are arranged on the carrier wheel 126. Another number of electron beam emitters is of course also possible. The carrier wheel 126 is rotatable around the axis c. Said axis c is directed in a direction transversal to the longitudinal axes *a* of the first electron beam emitters 10. The carrier wheel rotation is made clockwise in the figures, see arrow W. The carrier wheel 126 is also provided with gripping means 128 adapted to grip the packaging container 12 and displace it in relation to the first electron beam emitter 10 to perform inside surface sterilization of the packaging container 12, which will be further described below. The gripping means 128 grips the packaging container 12 near the open end 34 thereof. The gripping means 128 comprises shafts 128a radially attached to the carrier wheel 126. Gripping elements 128b are slidably attached on the shafts, said gripping elements 128b being able to grip the packaging container 12, either at least partly around the sleeve near the opening 34 or at the axial end of the sleeve near the opening 34, and displace it along the shafts 128a. The shafts 128a are provided parallel to the longitudinal axis of the first electron beam emitter and in the vicinity of the first electron beam emitter. The gripping elements 128b are displaceable along the shafts 128a by means of for example servo motors or cam curves arranged near on in the centre of the carrier wheel 126.

The packaging containers 12 are carried to the sterilization chamber 60 by a partly shown in-feed conveying means 130. The in-feed conveying means 130 is in this example a conventional conveyor belt, rotating intermittently or continuously, carrying the packaging containers 12 with the longitudinal axes of the packaging containers aligned with the horizontal direction. At an in-feed position 68 one packaging container 12 and one first electron beam emitter 10 in the carrier wheel 126 are aligned in front of each other, i.e. their longitudinal axes are aligned and coincide, see the visible line *l* in Fig. 11b. The gripping means 128 of the carrier wheel 126 picks the packaging container 12 from the in-feed conveying means 130 and transfers it over to the first electron beam emitter 10. The carrier wheel 126 is rotating continuously. The packaging container 10 is there started being displaced towards the first electron beam emitter 10. The first electron beam emitter 10 is aligned with the opening 34 of the packaging container 12 and hence the electron beam emitter 10 is received in the packaging container 12. As soon as the packaging container 12 comes into the electron cloud III of the first electron beam emitter sterilization of the interior of the packaging container 12 is commenced. Somewhere between the in-feed position 68 and an out-feed position the packaging container 12 has been displaced, by the gripping means 128, such that the packaging container 12 is fully engaged with the first electron beam emitter 10. The fully engaged second position is shown in Fig. 1a. In that position the innermost area of the packaging container 12 may be sterilized, in this case a top portion 70 of the packaging container 12.

The packaging container 12 is carried in the carrier wheel 126 for approximately 270 degrees before ending up at an out-feed position, being the interface region 64, i.e. the entrance to the aseptic chamber 62. In this seventh embodiment the interface region 64 comprises two second electron beam emitters 36 arranged with their electron exit windows 40 facing each other and with a passage for packaging containers 12 there between. The electron exit windows 40 are arranged either as shown in Fig. 5a or as shown in Fig. 6c. However, they are arranged with their longitudinal axes in parallel with each other and with their longitudinal axes inclined an angle β in relation to the vertical direction, see Fig. 11b. The longitudinal axis of one second electron beam emitter is illustrated by the dashed-dotted line b. Since the second electron beam emitters being inclined, with regard to the vertical direction, also their electron exit windows 40 are inclined, resulting in an inclined interface region 64.

In this embodiment the carrier wheel 126 is arranged above the interface region 64 and the aseptic chamber 62. Further, the out-feed position is in this embodiment a position, denoted 132, in which the first electron beam emitter 10 is positioned with its longitudinal axis, represented by the dash-dotted line *a*, aligned with the vertical direction and in which the first electron beam emitter is arranged right above, or slightly in, the interface region 64. The dash-dotted line *a* is shown visible, for the sake of understanding, but is should of course be understood that the axis extends through the centre of the respective first electron beam emitter.

Just before the packaging container reaches the out-feed position 132 the packaging container is held at the outermost end of the first electron beam emitter 10, i.e. the opening 34 in the packaging container 12 is situated near the electron exit window 20.

Due to the spokes arrangement of the first electron beam emitters 10 and the inclined second electron beam emitters 36 the cap of the packaging container 12 as well as a small portion of the packaging container top portion 70 will be the first areas of the packaging container entering the electron cloud I of the second electron beam emitters 36. This is made just before the packaging container 12 reaches the out-feed position 132.

At the out-feed position 132 the packaging container 12 is pushed off the first electron beam emitter 10 by the gripping means 128. During this pushing action, being made in the vertical direction, the gripping means 128 enters the interface region 64 and reaches approximately half way through it. However, it will never enter the aseptic chamber 62. Instead, the packaging container 12 is received by a second conveying means 134 inside the aseptic chamber 62. Hence, the gripping means 128, being a part of the first conveying means, and the second conveying means 134 are separately arranged one on each side of the interface region 64.

When the packaging container 12 is pushed through the interface region 64, and thereby through the electron cloud I of the second electron beam emitters 36, its outside surface is sterilized. Further, when the gripping means 128 of the carrier wheel 126 releases the packaging container 12 the electron cloud III emitted from the first electron beam emitter 10 is partly overlapping the electron cloud I emitted from the second electron beam emitters 36. By this any still unsterile area near the opening 34 of the packaging container 12, including the area where the gripping means 128 was gripping, is sterilized. A fully sterilized packaging container 12 enters the aseptic chamber 62.

As can be seen in the figures walls 136 surround and encapsulate the aseptic chamber 62. The only passage between the sterilization chamber 60 and the aseptic chamber 62 is through the interface region 64. The walls 136 are shown in Fig. 11a only.

The second conveying means 134 carries each packaging container 12 by gripping it around the top and/or the cap. The second conveying means 134 is adapted to move the packaging containers 12 by a horizontal movement and can be of any conventional type. The horizontal movement is shown as arrow H in Fig. 11b.

As has been touched upon above, and with specific reference to Fig. 11b, the carrier wheel 126 with its first electron beam emitters 10 are arranged such in relation to the second electron beam emitters 36 that when the carrier wheel 126 rotates the outermost portion of the first electron beam emitter, present at the out-feed position 132, enters the inclined interface region 64, but does not enter the aseptic chamber 62. The centre of the carrier wheel 126 is positioned approximately straight above the centre of the interface region 64.

The horizontal and vertical direction has been referred to in this embodiment. The two directions are transversal to each other and are shown in Fig. 11b. Line 1 is aligned with the horizontal direction as well as the packaging container transport direction in the second conveying means 134. The longitudinal axis of the first electron beam emitter positioned in the out-feed position is aligned with the vertical direction.

In the figures the sterilization chamber 60 has been shown as being without chamber walls. It should however be understood that the sterilization chamber 60 comprises walls such that the environment in the sterilization chamber can be controlled, especially such that sterile air flows, ozone and radiation can be controlled.

In the embodiment of the invention the second electron beam emitters 36 are shown in an inclined position. In other embodiments other positions might be more suitable depending on for example the size, type and energy of the electron beam emitters, the speed of the filling machine, the size and shape of the electron exit windows, the available space provided in the machine and the type of second conveying means. For example the carrier wheel 126 may be combined with an interface region 64 aligned to the vertical direction; i.e. the second electron beam emitters shown may have their longitudinal axes aligned with the vertical direction.

In the embodiment of the invention the carrier wheel is rotating continuously, however it could instead be rotating in an intermittent manner. In such case it is preferred if the second conveying means, in the aseptic chamber, is moving intermittently as well.

Although the present invention has been described with respect to several embodiments, it is to be understood that various modifications and changes may be made without departing from the object and scope of the invention as defined in the appended claims.

In the Figure 5a embodiment two second electron beam emitters are shown opposite each other. In an alternative embodiment there is only one second electron beam emitter, and the packaging container is made to rotate approximately one round around its own longitudinal axis when passing through the interface region. In order to ensure an aseptic lock, the opening between the sterilization chamber and the aseptic chamber should not be larger than that the electron cloud, generated by the single second electron beam emitter, can cover the opening.

The sterilization device has been described and illustrated in a schematic way in the above described embodiments and in the drawings. Only parts of the sterilization device being involved in the invention has been described, but it is to be understood that the sterilization device comprises also additional parts such as drive units for driving the first and second conveying means, and irradiation shielding enclosing the sterilization device for securing that electrons and x-rays are not spread to the environment outside of the device.

In the embodiments the packaging containers are carton bottles. However, it is easily realized that the invention can be applied also for other types of bottles, for example PET-bottles, for which sterilization and filling is made through the spout/neck. Similarly, the present invention is of course also applicable to sterilization of plastic bottle pre-forms, e.g. PET pre-forms. Sterilization of pre-forms is made before they are blow-moulded into a finished PET bottle. Hence, the interpretation of the word "packaging container" should in this regard be broad and comprises different types of containers such as for example carton-based packaging containers, plastic bottles and plastic pre-forms.

Although the embodiments have been described in relation to packaging containers for packaging of food products it should be realized that the packaging containers can be aimed for other types of products such as for example medical devices and instruments.

In the described embodiments the first chamber has been described as a sterilization chamber and the second chamber as an aseptic chamber, both aimed for aseptic packaging. In another embodiment, in which the packages are disinfected or hygienically treated, not aiming for aseptic level, i.e. not aiming for commercial sterility, the first chamber can be less clean than the second chamber, and the second chamber not being clean to an aseptic level. In such an embodiment the dose applied by the electron beam emitters is normally lower than the dose used for aseptic packaging. The emitters are then not sterilizing the packaging containers, but merely irradiating them to a desired level.

In the embodiments shown the first electron beam emitters are stationary in the vertical direction, i.e. the relative movement between the first electron beam emitter and and the packaging container is performed by the packaging container only. However, in an alternative embodiment the electron beam emitter 10 is moved and the packaging container is stationary in the vertical direction. The electron beam emitter is hence lowered into the open end of the packaging container.

## Claims

1. Irradiation device for irradiating packaging containers (12) with electron beams, said irradiation device comprising
at least one first electron beam emitter (10) adapted for irradiation of at least the interior of the packaging container (12),
at least one second electron beam emitter (36) adapted for irradiation of at least the exterior of said packaging container (12),
a first chamber (60) in which the first electron beam emitter (10) is adapted to irradiate the interior of the packaging container, the first chamber (60) comprising at least first conveying means, and
wherein
the at least one second electron beam emitter (36) is positioned such that an electron exit window thereof is facing an interface region (64; 64'), said interface region (64; 64') forming an opening from the first chamber (60) to a second chamber (62), and wherein an electron cloud (I; II) emitted from the at least one second electron beam emitter (36) during operation is adapted to form an irradiation lock at least covering the interface region (64; 64'), **characterised in that**
the first conveying means comprises a carrier wheel (126) to which more than one first electron beam emitter (10) are arranged as spokes, having their respective longitudinal axis radially directed in relation to a centre of the carrier wheel (126) and each having an electron exit window (20) arranged transverse to the longitudinal axis in the outer end of each spoke.

2. Irradiation device according to claim 1, wherein an electron cloud (III) emitted from the first electron beam emitter (10), upon finalisation of the interior irradiation of the packaging container (12), is adapted to be partly overlapping the electron cloud (I; II) emitted from the at least one second electron beam emitter (36), said electron clouds (I, II, III) together forming a combined electron cloud, and wherein an opening (34) of the packaging container is at least temporarily positioned within the combined cloud.

3. Irradiation device according to any of the preceding claims, wherein the second chamber (62) comprises at least second conveying means, and that the first and second conveying means are separately arranged one on each side of the interface region (64; 64'), and wherein the first conveying means is adapted to push the packaging container at least partly through the irradiation lock in the interface region (64; 64'), and release the packaging container when it has been received by the second conveying means on the other side of the irradiation lock.

4. Irradiation device according to any of the preceding claims, wherein the first electron beam emitter (10) and the packaging container (12) are adapted to perform a mutual relative movement during which the interior irradiation of the packaging container (12) takes place.

5. Irradiation device according to claim 4, wherein the first conveying means is adapted to displace the packaging container (12) in relation to the first electron beam emitter (10) between a first position in which the packaging container (12) and the first electron beam emitter (10) are not engaged with each other and a second position in which the packaging container (12) and the first electron beam emitter (10) are fully engaged with each other.

6. Irradiation device according to any of the preceding claims, wherein it comprises two second electron beam emitters (36), arranged opposite each other with their electron exit windows (40) facing each other and the interface region (64; 64'), in such a way that the packaging containers (12) can pass in between them.

7. Irradiation device according to any of the claims 3-6, wherein the first electron beam emitter (10) is arranged in the first conveying means, and wherein the first conveying means is adapted to convey the packaging container (12) synchronously with the first electron beam emitter (10), keeping a longitudinal axis of the first electron beam emitter (10) aligned with a longitudinal axis of the packaging container (12).

8. Irradiation device according to claim 7, wherein the first conveying means is adapted to displace the packaging container (12) from a first chamber in-feed position (68; 78) to an out-feed position, the out-feed position being the interface region (64; 64'), and that the first electron beam emitter (10) is adapted to irradiate the interior of the packaging container (12) at least during a portion of the displacement between the first chamber in-feed position (68; 78) to the out-feed position.

9. Irradiation device according to any of the preceding claims, wherein said at least one second electron beam emitter (36) being adapted to irradiate the exterior of the packaging container (12) when passing the packaging container from the first chamber (60) to the second chamber (62), through the interface region (64; 64'), such that the packaging container reaching the second chamber (62) is fully irradiated on its exterior.

10. Irradiation device according to any of the preceding claims, wherein the irradiation device is a sterilization device for sterilizing packaging containers, wherein the irradiation lock is an aseptic lock and wherein the second chamber is an aseptic chamber (62).

11. Irradiation device according to claim 10, wherein the aseptic chamber (62) comprises stations in which the packaging container is adapted to be filled with product and sealed, and wherein the first chamber (60) is a sterilization chamber.

12. Method of irradiating packaging containers (12) with electron beams, said method comprising
irradiating at least the interior of the packaging container (12) with a first electron beam emitter (10) arranged in a first chamber (60), the first chamber (60) comprising at least first conveying means,
irradiating at least the exterior of the packaging container (12) with at least one second electron beam emitter (36), and
wherein
the step of irradiating the exterior of the packaging container is performed in an interface region (64), said interface region (64; 64') forming an opening from the first chamber (60) to a second chamber (62), and wherein an electron cloud (I; II) emitted from the at least one second electron beam emitter (36) during operation is adapted to form an irradiation lock at least covering the interface region (64; 64'), **characterised in that** the first conveying means comprises a carrier wheel (126) to which more than one first electron beam emitter (10) are arranged as spokes, having their respective longitudinal axis radially directed in relation to a centre of the carrier wheel (126) and each having an electron exit window (20) arranged transverse to the longitudinal axis in the outer end of each spoke.

13. Method according to claim 12, wherein the method comprises the step of finalizing the interior irradiation such that an electron cloud (III) emitted from the first electron beam emitter (10) partly overlaps the electron cloud (I; II) emitted from the at least one second electron beam emitter (36), said electron clouds (I, II, III) together forming a combined electron cloud, and wherein it comprises the step of at least temporarily positioning an opening (34) of the packaging container within the combined cloud.

14. Method according to any of the preceding claims 12-13, wherein the steps of irradiating the interior and exterior of the packaging container are steps of sterilizing the interior and exterior of the packaging container.

## Patentansprüche

1. Bestrahlungsvorrichtung zur Bestrahlung von Verpackungsbehältern (12) mit Elektronenstrahlen, wobei die Bestrahlungsvorrichtung umfasst:
mindestens einen ersten Elektronenstrahl-Emitter (10), der zur Bestrahlung mindestens des Inneren des Verpackungsbehälters (12) geeignet ist,
mindestens einen zweiten Elektronenstrahl-Emitter (36), der zur Bestrahlung mindestens des Äußeren des Verpackungsbehälters (12) geeignet ist,
eine erste Kammer (60), in welcher der erste Elektronenstrahl-Emitter (10) geeignet ist, das Innere des Verpackungsbehälters zu bestrahlen, wobei die erste Kammer (60) mindestens ein erstes Fördermittel umfasst, und
wobei der mindestens eine zweite Elektronenstrahl-Emitter (36) derart positioniert ist, dass ein Elektronenaustrittsfenster davon einer Grenzflächenzone (64; 64') zugewandt ist, wobei die Grenzflächenzone (64; 64') eine Öffnung von der ersten Kammer (60) zu einer zweiten Kammer (62) bildet, und wobei eine Elektronenwolke (I; II), die von dem mindestens einen zweiten Elektronenstrahl-Emitter (36) während des Betriebs emittiert wird, geeignet ist, eine Bestrahlungssperre zu bilden, die mindestens die Grenzflächenzone (64; 64') abdeckt;
**dadurch gekennzeichnet, dass**:
das erste Fördermittel ein Trägerrad (126) umfasst, an dem mehr als ein erster Elektronenstrahl-Emitter (10) als Speichen angeordnet sind, wobei ihre jeweilige Längsachse radial in Bezug auf ein Zentrum des Trägerrads (126) ausgerichtet ist, und die jeweils ein Elektronenaustrittsfenster (20) aufweisen, das quer zu der Längsachse in dem äußeren Ende jeder Speiche angeordnet ist.

2. Bestrahlungsvorrichtung nach Anspruch 1,
wobei eine Elektronenwolke (III), die von dem ersten Elektronenstrahl-Emitter (10) emittiert wird, bei der Beendigung der inneren Bestrahlung des Verpackungsbehälters (12), geeignet ist, die Elektronenwolke (I; II) teilweise zu überlappen, die von dem mindestens einen zweiten Elektronenstrahl-Emitter (36) emittiert wird, wobei die Elektronenwolken (I, II, III) gemeinsam eine kombinierte Elektronenwolke bilden, und wobei eine Öffnung (34) des Verpackungsbehälters mindestens temporär innerhalb der kombinierten Wolke positioniert ist.

3. Bestrahlungsvorrichtung nach einem der vorhergehenden Ansprüche,
wobei die zweite Kammer (62) mindestens ein zweites Fördermittel umfasst, und dass das erste und zweite Fördermittel getrennt auf jeder Seite der Grenzflächenzone (64; 64') angeordnet sind, und wobei das erste Fördermittel geeignet ist, den Verpackungsbehälter mindestens teilweise durch die Bestrahlungssperre in der Grenzflächenzone (64; 64') zu schieben, und den Verpackungsbehälter freizugeben, wenn dieser von dem zweiten Fördermittel auf der anderen Seite der Bestrahlungssperre aufgenommen wurde.

4. Bestrahlungsvorrichtung nach einem der vorhergehenden Ansprüche,
wobei der erste Elektronenstrahl-Emitter (10) und der Verpackungsbehälter (12) geeignet sind, eine gegenseitige relative Bewegung vorzunehmen, während welcher die innere Bestrahlung des Verpackungsbehälters (12) stattfindet.

5. Bestrahlungsvorrichtung nach Anspruch 4,
wobei das erste Fördermittel geeignet ist, den Verpackungsbehälter (12) in Bezug auf den ersten Elektronenstrahl-Emitter (10) zwischen einer ersten Position, in welcher der Verpackungsbehälter (12) und der erste Elektronenstrahl-Emitter (10) nicht miteinander in Eingriff stehen, und einer zweiten Position, in welcher der Verpackungsbehälter (12) und der erste Elektronenstrahl-Emitter (10) vollständig miteinander in Eingriff stehen, zu verschieben.

6. Bestrahlungsvorrichtung nach einem der vorhergehenden Ansprüche,
wobei diese zwei zweite Elektronenstrahl-Emitter (36) umfasst, die einander gegenüber angeordnet sind, wobei ihre Elektronenaustrittsfenster (40) einander und der Grenzflächenzone (64; 64') derart zugewandt sind, dass der Verpackungsbehälter (12) zwischen diesen hindurchgehen kann.

7. Bestrahlungsvorrichtung nach einem der Ansprüche 3 bis 6,
wobei der erste Elektronenstrahl-Emitter (10) in dem ersten Fördermittel angeordnet ist, und wobei das erste Fördermittel geeignet ist, den Verpackungsbehälter (12) synchron mit dem ersten Elektronenstrahl-Emitter (10) zu befördern, wobei eine Längsachse des ersten Elektronenstrahl-Emitters (10) mit einer Längsachse des Verpackungsbehälters (12) ausgerichtet bleibt.

8. Bestrahlungsvorrichtung nach Anspruch 7,
wobei das erste Fördermittel geeignet ist, den Verpackungsbehälter (12) von einer ersten Kammer-Einführposition (68; 78) zu einer Ausführposition zu verschieben, wobei die Ausführposition die Grenzflächenzone (64; 64') ist, und dass der erste Elektronstrahl-Emitter (10) geeignet ist, das Innere des Verpackungsbehälters (12) mindestens während eines Abschnitts der Verschiebung zwischen der ersten Kammer-Einführposition (68; 78) zu der Ausführposition zu bestrahlen.

9. Bestrahlungsvorrichtung nach einem der vorhergehenden Ansprüche,
wobei der mindestens eine zweite Elektronstrahl-Emitter (36) geeignet ist, das Äußere des Verpackungsbehälters (12) zu bestrahlen, wenn der Verpackungsbehälter von der ersten Kammer (60) zu der zweiten Kammer (62) durch die Grenzflächenzone (64; 64') geführt wird, so dass der Verpackungsbehälter, der die zweite Kammer (62) erreicht, an seiner Außenseite vollständig bestrahlt wird.

10. Bestrahlungsvorrichtung nach einem der vorhergehenden Ansprüche,
wobei die Bestrahlungsvorrichtung eine Sterilisierungsvorrichtung zur Sterilisierung von Verpackungsbehältern ist, wobei die Bestrahlungssperre eine aseptische Sperre ist, und wobei die zweite Kammer eine aseptische Kammer (62) ist.

11. Bestrahlungsvorrichtung nach Anspruch 10,
wobei die aseptische Kammer (62) Stationen umfasst, in denen der Verpackungsbehälter geeignet ist, mit Produkt gefüllt zu werden und versiegelt zu werden, und wobei die erste Kammer (60) eine Sterilisierungskammer ist.

12. Verfahren zur Bestrahlung von Verpackungsbehältern (12) mit Elektronenstrahlen, wobei das Verfahren umfasst:
Bestrahlen mindestens des Inneren des Verpackungsbehälters (12) mit einem ersten Elektronenstrahl-Emitter (10), der in einer ersten Kammer (60) angeordnet ist, wobei die erste Kammer (60) mindestens ein erstes Fördermittel umfasst, Bestrahlen mindestens des Äußeren des Verpackungsbehälters (12) mit mindestens einem zweiten Elektronenstrahl-Emitter (36), und
wobei der Schritt des Bestrahlens des Äußeren des Verpackungsbehälters in einer Grenzflächenzone (64) vorgenommen wird, wobei die Grenzflächenzone (64; 64') eine Öffnung von der ersten Kammer (60) zu einer zweiten Kammer (62) bildet, und wobei eine Elektronenwolke (I; II), die von dem mindestens einen zweiten Elektronenstrahl-Emitter (36) während des Betriebs emittiert wird, geeignet ist, eine Bestrahlungssperre zu bilden, die mindestens die Grenzflächenzone (64; 64') abdeckt;
**dadurch gekennzeichnet, dass**:
das erste Fördermittel ein Trägerrad (126) umfasst, an dem mehr als ein erster Elektronenstrahl-Emitter (10) als Speichen angeordnet sind, wobei ihre jeweilige Längsachse radial in Bezug auf ein Zentrum des Trägerrads (126) ausgerichtet ist, und die jeweils ein Elektronenaustrittsfenster (20) aufweisen, das quer zu der Längsachse in dem äußeren Ende jeder Speiche angeordnet ist.

13. Verfahren nach Anspruch 12,
wobei das Verfahren den Schritt des Beendens der inneren Bestrahlung umfasst, so dass eine Elektronenwolke (III), die von dem ersten Elektronenstrahl-Emitter (10) emittiert wird, die Elektronenwolke (I; II) teilweise überlappt, die von dem mindestens einen zweiten Elektronenstrahl-Emitter (36) emittiert wird, wobei die Elektronenwolken (I, II, III) gemeinsam eine kombinierte Elektronenwolke bilden, und wobei dieses den Schritt des mindestens temporären Positionierens einer Öffnung (34) des Verpackungsbehälters innerhalb der kombinierten Wolke umfasst.

14. Verfahren nach einem der vorhergehenden Ansprüche 12 bis 13,
wobei die Schritte des Bestrahlens des Inneren und Äußeren des Verpackungsbehälters Schritte des Sterilisierens des Inneren und Äußeren des Verpackungsbehälters sind.

## Revendications

1. Dispositif d'irradiation pour irradier des conteneurs de conditionnement (12) avec des faisceaux d'électron, ledit dispositif d'irradiation comprenant
au moins un premier émetteur de faisceau d'électron (10) conçu pour l'irradiation d'au moins l'intérieur du conteneur de conditionnement (12),
au moins un second émetteur de faisceau d'électron (36) conçu pour l'irradiation d'au moins l'extérieur dudit conteneur de conditionnement (12),
une première chambre (60) dans laquelle le premier émetteur de faisceau d'électron (10) est conçu pour irradier l'intérieur du conteneur de conditionnement, la première chambre (60) comprenant au moins un premier moyen de transport et dans lequel le ou les seconds émetteurs de faisceau d'électron (36) sont positionnés de telle sorte qu'une fenêtre de sortie d'électron de ce dernier ou de ces derniers soit orientée vers une région d'interface (64 ; 64"), ladite région d'interface (64 ; 64') formant une ouverture depuis la première chambre (60) jusqu'à une seconde chambre (62) et dans lequel un nuage d'électrons (I ; II) émis depuis le ou les seconds émetteurs de faisceau d'électron (36) pendant le fonctionnement est conçu pour former un verrou d'irradiation couvrant au moins la région d'interface (64 ; 64'), **caractérisé en ce que** le premier moyen de transport comprend une roue de support (126) à laquelle au moins deux premiers émetteurs de faisceau d'électron (10) sont agencés sous la forme de rayons, ayant leur axe longitudinal respectif dirigé radialement par rapport au centre de la roue de support (126) et ayant chacun une fenêtre de sortie d'électron (20) agencée transversalement à l'axe longitudinal dans l'extrémité externe de chaque rayon.

2. Dispositif d'irradiation selon la revendication 1, dans lequel un nuage d'électrons (III) émis depuis le premier émetteur de faisceau d'électron (10), lors de la finalisation de l'irradiation intérieure du conteneur de conditionnement (12), est conçu pour recouvrir partiellement le nuage d'électrons (I ; II) émis depuis le ou les seconds émetteurs de faisceau d'électron (36), lesdits nuages d'électrons (I, II, III) formant ensemble un nuage d'électrons combiné, et dans lequel une ouverture (34) du conteneur de conditionnement est au moins temporairement positionnée à l'intérieur du nuage combiné.

3. Dispositif d'irradiation selon l'une quelconque des revendications précédentes, dans lequel la seconde chambre (62) comprend au moins un second moyen de transport et que les premier et second moyens de transport sont agencés de manière séparée un sur chaque côté de la région d'interface (64 ; 64') et dans lequel le premier moyen de transport est conçu pour pousser le conteneur de conditionnement au moins partiellement à travers le verrou d'irradiation dans la région d'interface (64 ; 64') et pour libérer le conteneur de conditionnement lorsqu'il a été reçu par le second moyen de transport sur l'autre côté du verrou d'irradiation.

4. Dispositif d'irradiation selon l'une quelconque des revendications précédentes, dans lequel le premier émetteur de faisceau d'électron (10) et le conteneur de conditionnement (12) sont conçus pour effectuer un mouvement relatif mutuel pendant lequel l'irradiation intérieure du conteneur de conditionnement (12) a lieu.

5. Dispositif d'irradiation selon la revendication 4, dans lequel le premier moyen de transport est conçu pour déplacer le conteneur de conditionnement (12) par rapport au premier émetteur de faisceau d'électron (10) entre une première position dans laquelle le conteneur de conditionnement (12) et le premier émetteur de faisceau d'électron (10) ne sont pas en prise l'un avec l'autre, et une seconde position dans laquelle le conteneur de conditionnement (12) et le premier émetteur de faisceau d'électron (10) sont complètement en prise l'un avec l'autre.

6. Dispositif d'irradiation selon l'une quelconque des revendications précédentes, **caractérisé en ce qu'**il comprend deux seconds émetteurs de faisceau d'électron (36), agencés à l'opposé l'un de l'autre avec leurs fenêtres de sortie d'électron (40) orientées l'une vers l'autre et vers la région d'interface (64 ; 64') de telle manière que les conteneurs de conditionnement (12) puissent passer entre elles.

7. Dispositif d'irradiation selon l'une quelconque des revendications 3 à 6, dans lequel le premier émetteur de faisceau d'électron (10) est agencé dans le premier moyen de transport et dans lequel le premier moyen de transport est conçu pour transporter le conteneur de conditionnement (12) de manière synchrone avec le premier émetteur de faisceau d'électron (10), gardant un axe longitudinal du premier émetteur de faisceau d'électron (10) aligné sur un axe longitudinal du conteneur de conditionnement (12).

8. Dispositif d'irradiation selon la revendication 7, dans lequel le premier moyen de transport est conçu pour déplacer le conteneur de conditionnement (12) depuis une première position d'alimentation de chambre (68 ; 78) jusqu'à une position d'évacuation, la position d'évacuation étant la région d'interface (64 ; 64') et le premier émetteur de faisceau d'électron (10) est conçu pour irradier l'intérieur du conteneur de conditionnement (12) au moins pendant une partie du déplacement entre la première position d'alimentation de chambre (68 ; 78) et la position d'évacuation.

9. Dispositif d'irradiation selon l'une quelconque des revendications précédentes, dans lequel ledit ou lesdits seconds émetteurs de faisceau d'électron (36) sont conçus pour irradier l'extérieur du conteneur de conditionnement (12) lors du passage du conteneur de conditionnement de la première chambre (60) à la seconde chambre (62), à travers la région d'interface (64 ; 64') de telle sorte que le conteneur de conditionnement atteignant la seconde chambre (62) soit complètement irradié sur son extérieur.

10. Dispositif d'irradiation selon l'une quelconque des revendications précédentes, le dispositif d'irradiation étant un dispositif de stérilisation pour stériliser des conteneurs de conditionnement, dans lequel le verrou d'irradiation est un verrou aseptique et dans lequel la seconde chambre est une chambre aseptique.

11. Dispositif d'irradiation selon la revendication 10, dans lequel la chambre aseptique (62) comprend des postes dans lesquels le conteneur de conditionnement est conçu pour être rempli d'un produit et scellé et dans lequel la première chambre (60) est une chambre de stérilisation.

12. Procédé d'irradiation pour irradier des conteneurs de conditionnement (12) avec des faisceaux d'électron, ledit procédé consistant :
à irradier au moins l'intérieur du conteneur de conditionnement (12) avec un premier émetteur de faisceau d'électron (10) agencé dans une première chambre (60), la première chambre (60) comprenant au moins un premier moyen de transport,
à irradier au moins l'extérieur du conteneur de conditionnement (12) avec au moins un second émetteur de faisceau d'électron (36), et
dans lequel
l'étape d'irradiation de l'extérieur du conteneur de conditionnement est réalisée dans une région d'interface (64), ladite région d'interface (64 ; 64') formant une ouverture depuis la première chambre (60) jusqu'à une seconde chambre (62) et dans lequel un nuage d'électrons (I ; II) émis depuis le ou les seconds émetteurs de faisceau d'électron (36) pendant le fonctionnement est conçu pour former un verrou d'irradiation couvrant au moins la région d'interface (64 ; 64'), **caractérisé en ce que**
le premier moyen de transport comprend une roue de support (126) à laquelle au moins deux premiers émetteurs de faisceau d'électron (10) sont agencés sous la forme de rayons, ayant leur axe longitudinal respectif dirigé radialement par rapport au centre de la roue de support (126) et ayant chacun une fenêtre de sortie d'électron (20) agencée transversalement à l'axe longitudinal dans l'extrémité externe de chaque rayon.

13. Procédé selon la revendication 12, le procédé comprenant l'étape de finalisation de l'irradiation intérieure de telle sorte qu'un nuage d'électrons (III) émis depuis le premier émetteur de faisceau d'électron (10) recouvre partiellement le nuage d'électrons (I ; II) émis depuis le ou les seconds émetteurs de faisceau d'électron (36), lesdits nuages d'électrons (I, II, III) formant ensemble un nuage d'électrons combiné, et **caractérisé en ce qu'**il comprend l'étape consistant à positionner au moins temporairement une ouverture (34) du conteneur de conditionnement à l'intérieur du nuage combiné.

14. Procédé selon l'une quelconque des revendications 12 à 13, dans lequel les étapes d'irradiation de l'intérieur et de l'extérieur du conteneur de conditionnement sont des étapes de stérilisation de l'intérieur et de l'extérieur du conteneur de conditionnement.
